# EUROPEAN PATENT APPLICATION

(11) **EP 4 083 024 A1**
(43) Date of publication of application: **02.11.2022**
(21) Application number: 20892964.6
(22) Date of filing: 25.11.2020
(51) Int. Cl.: C07D 237/14, C07D 237/16, C07D 237/18, C07D 403/10, C07D 403/12, A61K 31/501, A61K 31/53, A61P 5/16

(54) **1,2,4-TRIAZINE-3,5-DIONE COMPOUND, PREPARATION METHOD THEREFOR, AND APPLICATION THEREOF**

(30) Priority: 26.11.2019 CN 201911177482
(71) Applicant: KPC Pharmaceuticals, Inc., Kunming, Yunnan 650106 (CN); Shanghai Kunheng Pharma-Tech Co., Ltd., Shanghai 201210 (CN)
(72) Inventor: LV, Hejun, Kunming, Yunnan 650106 (CN); WANG, Peng, Kunming, Yunnan 650106 (CN); LIANG, Hui, Kunming, Yunnan 650106 (CN); GUO, Fei, Kunming, Yunnan 650106 (CN); YE, Wenwu, Kunming, Yunnan 650106 (CN); REN, Lifeng, Kunming, Yunnan 650106 (CN); LIU, Jun, Kunming, Yunnan 650106 (CN)
(74) Representative: Lavoix
(86) International application number: PCT/CN2020/131367
(87) International publication number: WO 2021/104288

(57) **Abstract**

The present invention provides a 1,2,4-triazine-3,5-dione compound, a preparation method therefor, and an application thereof. The 1,2,4-triazine-3,5-dione compound having a structure represented by formula (I) provided by present invention has a selective agonistic effect on THRβ, and the activity, selectivity, and metabolic stability thereof have significant advantages over disclosed compounds, and can function as a therapeutic and/or preventive drug for thyroid hormone receptor related diseases, comprising, but not limited to, obesity, diabetes, hypercholesterolemia, hyperlipidemia, hypertriglyceridemia, liver steatosis, non-alcoholic fatty liver disease, nonalcoholic steatohepatitis, familial hypercholesterolemia, dyslipidemia, atherosclerosis, hypothyroidism, and thyroid cancer.

## Description

This application claims the priority of Chinese Patent Application No. 201911177482.7, filed with the China National Intellectual Property Administration on November 26, 2019, and titled with "1,2,4-TRIAZINE-3,5-DIONE COMPOUND, PREPARATION METHOD THEREFOR, AND APPLICATION THEREOF", which are hereby incorporated by reference in entirety.

### FIELD

The present disclosure relates to the technical field of medicine, and specifically relates to a 1,2,4-triazine-3,5-dione compound, a preparation method therefor, and an application thereof.

### BACKGROUND

Thyroid hormones play a key role in normal growth and development of the body and in maintaining metabolic balance (Physiological Reviews 2001, 81(3), 1097-1126.). Thyroid hormones are produced by the thyroid and are secreted into the circulatory system (hypothalamic/pituitary/thyroid system) in two different forms, T4 and T3, with T4 being the predominant form secreted by the thyroid and T3 being the more physiologically active form. T4 is converted to T3 by tissue-specific deiodinase, which is present in all tissues, but mainly in liver and kidney tissues.

The physiological activity of thyroid hormones is mainly mediated by thyroid hormone receptors (THRs) (Endocrine Reviews 1993, 14, 348-399.). THR belongs to the nuclear receptor superfamily, and forms a heterodimer with a retinoid receptor that acts as a ligand-induced transcription factor. THR has a ligand-binding domain, a DNA-binding domain, and an amino-terminal domain, and regulates gene expression through interactions with DNA-responsive elements and with various nuclear co-activators and co-repressors. THR is encoded by the expression α and β of different genes located on human chromosomes 17 and 3. Different protein isoforms are generated by selective splicing of the primary transcript. Each gene produces two isoforms, namely THRα1, THRα2, THRβ1 and THRβ2. THRβ1 and THRβ2 are obtained by differential expression from promoters, and the two isoforms differ only at the amino terminus. THRα1 and THRα2 result from differential splicing of pre-mRNAs, and mainly differ at the carboxy terminus. THRα1, THRβ1 and THRβ2 can bind to thyroid hormones. Studies have shown that thyroid hormone receptor isoforms can differ in their contribution to specific physiological responses. THRβ1 plays an important role in the regulation of thyroid stimulating hormone and thyroid hormone in the liver, and THRβ2 plays a major role in the regulation of thyroid stimulating hormone. Studies have also shown that two isoforms of THR, α and β, coexist in the liver, of which THRβ, which is involved in lipid metabolism, accounts for 70-80%, and in the heart, THRα is associated with increased heartbeat and increased cardiac output (Endocrinology 2001, 142, 544; J. Biol. Chem. 1992, 267, 11794.).

Thyroid hormones are metabolized in target organs and excreted mainly in bile, and their physiological roles in mammals are mainly manifested in growth and differentiation and maintenance of life functions, such as heart rate, blood cholesterol and triglyceride concentrations, as well as control and regulation of systemic metabolism speed, body weight, etc. From a pathophysiological point of view, tachycardia, arrhythmia, heart failure, as well as fatigue, shortness of breath, sarcopenia, and osteoporosis are observed in hyperthyroidism such as Graves disease (Physiol. Rev. 2001, 81, 1097; J. Steroid. Biochem. Mol. Biol. 2001, 76, 31.).

The therapeutic use of thyroid hormone itself is limited by the adverse side effects associated with hyperthyroidism, especially cardiovascular toxicity. A thyroid hormone analog, if the adverse effects of hyperthyroidism and hypothyroidism can be avoided while maintaining the beneficial effects of thyroid hormones, may be used in the treatment responsive to diseases such as metabolic diseases including obesity, hyperlipidemia, hypercholesterolemia, diabetes and other conditions such as hepatic steatosis and nonalcoholic steatohepatitis (NASH), atherosclerosis, cardiovascular disease, hypothyroidism, thyroid cancer, thyroid disease and the like.

Thyroid hormone analogs structurally different from the compound of the present disclosure have been disclosed (Agricultural and Biol. Chem. 1974, 38(6), 1169; J. Med. Chem. 1989, 32, 320; J. Med. Chem. 2014, 57(10), 3912; WO2007009913; WO2010122980). Among them, WO2007009913 discloses a pyridazinone derivative, especially Example 8 (compound 31, i.e. MGL-3196), that has achieved good results as a thyroid hormone analog with THRβ selectivity and liver tissue selectivity and may be used to treat a variety of diseases. However, MGL-3196 still has problems such as insufficient activity and fast metabolism in the body. Therefore, it is necessary to continue to discover and develop new compounds with high activity, high selectivity and high metabolic stability, which have the beneficial effects of thyroid hormones and avoid adverse effects, for the treatment of diseases related to thyroid hormone receptors.

### SUMMARY

In view of this, the technical problem to be solved by the present disclosure is to provide a 1,2,4-triazine-3,5-dione compound, a preparation method therefor and an application thereof. The compound provided by the present disclosure not only has high activity and selectivity, but also has good metabolic stability.

Compared with the prior art, the present disclosure provides a 1,2,4-triazine-3,5-dione compound and pharmaceutically acceptable salt thereof. The compound with the structure represented by the formula (I) provided by the present disclosure has a selective agonistic effect on THRβ, and the activity, selectivity and metabolic stability thereof have significant advantages over the disclosed compounds, and can function as a therapeutic and/or preventive drug for thyroid hormone receptor-related diseases, including but not limited to, obesity, diabetes, hypercholesterolemia, hyperlipidemia, hypertriglyceridemia, liver steatosis, non-alcoholic fatty liver disease, nonalcoholic steatohepatitis, familial hypercholesterolemia, dyslipidemia, atherosclerosis, hypothyroidism, and thyroid cancer.

### DETAILED DESCRIPTION

The present disclosure provides a 1,2,4-triazine-3,5-dione compound and pharmaceutically acceptable salt thereof, wherein the 1,2,4-triazine-3,5-dione compound has a structure represented by formula (I), 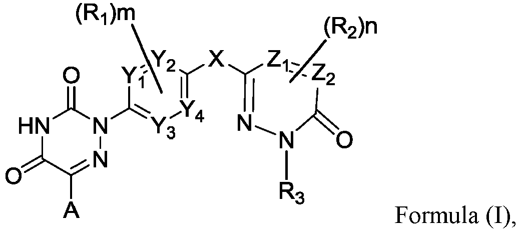
wherein, A is substituted or unsubstituted C1~C15 alkyl, substituted or unsubstituted C1~C15 alkoxy, substituted or unsubstituted C3~C18 cycloalkyl, substituted or unsubstituted C6~C20 aryl, substituted or unsubstituted C5~C20 heteroaryl, or substituted or unsubstituted C3~C18 heterocyclyl, hydroxyl or halogen;
X is substituted methylene, -O- or -S-,
Y₁, Y₂, Y₃, Y₄, Z₁ and Z₂ are independently selected from N and CR,
R₁, R₂, R₃ and R are independently selected from hydrogen, substituted or unsubstituted C1~C15 alkyl, substituted or unsubstituted C1~C15 alkoxy, substituted or unsubstituted C3~C18 cycloalkyl, and substituted or unsubstituted C2~C15 unsaturated hydrocarbyl, halogen or cyano; and
m and n are each an integer of 0-3.

According to the present disclosure, the substituent in the substituted C1~C15 alkyl, substituted C1~C15 alkoxy, substituted C3~C18 cycloalkyl, substituted C6~C20 aryl, substituted C5~C20 heteroaryl, substituted C3~C18 heterocyclyl and substituted C2~C15 unsaturated hydrocarbyl is hydroxyl, fluorine, chlorine, bromine, iodine or amino.

In the present disclosure, A is preferably substituted or unsubstituted C3~C10 alkyl, substituted or unsubstituted C3~C10 alkoxy, substituted or unsubstituted C5~C12 cycloalkyl, substituted or unsubstituted C10~C15 aryl, substituted or unsubstituted C8~C15 heteroaryl, or substituted or unsubstituted C5~C10 heterocyclyl, hydroxyl or halogen; more preferably methyl, ethyl, n-propyl, isopropyl, n-butyl, isobutyl, tert-butyl, n-pentyl, isopentyl, n-hexyl, n-heptyl, hydroxymethyl, hydroxyethyl, 2-hydroxypropyl, 3-hydroxypropyl, 2-hydroxybutyl, 2-hydroxypentyl, fluoromethyl, difluoromethyl, trifluoromethyl, 1-fluoroethyl, 1-fluoropropyl, 1-fluoroisopropyl, 1-fluorobutyl, 1,1-difluoroethyl, 1,1-difluoropropyl, 1,1-difluorobutyl, methoxy, ethoxy, propoxy, butoxy, pentoxy, cyclopropyl, cyclobutyl, cyclopentyl, cyclohexyl, phenyl, naphthyl, anthracenyl, pyridyl, pyrrolyl, furanyl, pyranyl, hydroxy, fluorine, chlorine, bromine or iodine.

According to the present disclosure, R is preferably hydrogen, substituted or unsubstituted C3~C10 alkyl, substituted or unsubstituted C3~C10 alkoxy, substituted or unsubstituted C5~C12 cycloalkyl, substituted or unsubstituted C5~C10 unsaturated hydrocarbyl, halogen or cyano, more preferably hydrogen, methyl, ethyl, n-propyl, isopropyl, n-butyl, isobutyl, tert-butyl, n-pentyl, isopentyl, n-hexyl, n-heptyl, hydroxymethyl, hydroxyethyl, 2-hydroxypropyl, 3-hydroxypropyl, 2-hydroxybutyl, 2-hydroxypentyl, fluoromethyl, difluoromethyl, trifluoromethyl, 1-fluoroethyl, 1-fluoropropyl, 1-fluoroisopropyl, 1-fluorobutyl, 1,1-difluoroethyl, 1,1-difluoropropyl, 1,1-difluorobutyl, methoxy, ethoxy, propoxy, butoxy, pentoxy, cyclopropyl, cyclobutyl, cyclopentyl, cyclohexyl, phenyl, naphthyl, anthracenyl, pyridyl, pyrrolyl, furyl, pyranyl, 1-methylvinyl, propenyl, butenyl, hydroxyl, fluorine, chlorine, bromine or iodine.

According to the present disclosure, R₁ is preferably hydrogen, substituted or unsubstituted C3~C10 alkyl, substituted or unsubstituted C3~C10 alkoxy, substituted or unsubstituted C5~C12 cycloalkyl, substituted or unsubstituted C5~C10 unsaturated hydrocarbyl, halogen or cyano, more preferably hydrogen, methyl, ethyl, n-propyl, isopropyl, n-butyl, isobutyl, tert-butyl, n-pentyl, isopentyl, n-hexyl, n-heptyl, hydroxymethyl, hydroxyethyl, 2-hydroxypropyl, 3-hydroxypropyl, 2-hydroxybutyl, 2-hydroxypentyl, fluoromethyl, difluoromethyl, trifluoromethyl, 1-fluoroethyl, 1-fluoropropyl, 1-fluoroisopropyl, 1-fluorobutyl, 1,1-difluoroethyl, 1,1-difluoropropyl, 1,1-difluorobutyl, methoxy, ethoxy, propoxy, butoxy, pentoxy, cyclopropyl, cyclobutyl, cyclopentyl, cyclohexyl, phenyl, naphthyl, anthracenyl, pyridyl, pyrrolyl, furyl, pyranyl, 1-methylvinyl, propenyl, butenyl, hydroxyl, fluorine, chlorine, bromine or iodine.

According to the present disclosure, R₂ is preferably hydrogen, substituted or unsubstituted C3~C10 alkyl, substituted or unsubstituted C3~C10 alkoxy, substituted or unsubstituted C5~C12 cycloalkyl, substituted or unsubstituted C5~C10 unsaturated hydrocarbyl, halogen or cyano, more preferably hydrogen, methyl, ethyl, n-propyl, isopropyl, n-butyl, isobutyl, tert-butyl, n-pentyl, isopentyl, n-hexyl, n-heptyl, hydroxymethyl, hydroxyethyl, 2-hydroxypropyl, 3-hydroxypropyl, 2-hydroxybutyl, 2-hydroxypentyl, fluoromethyl, difluoromethyl, trifluoromethyl, 1-fluoroethyl, 1-fluoropropyl, 1-fluoroisopropyl, 1-fluorobutyl, 1,1-difluoroethyl, 1,1-difluoropropyl, 1,1-difluorobutyl, methoxy, ethoxy, propoxy, butoxy, pentoxy, cyclopropyl, cyclobutyl, cyclopentyl, cyclohexyl, phenyl, naphthyl, anthracenyl, pyridyl, pyrrolyl, furyl, pyranyl, 1-methylvinyl, propenyl, butenyl, hydroxyl, fluorine, chlorine, bromine or iodine.

According to the present disclosure, R₃ is preferably hydrogen, substituted or unsubstituted C3~C10 alkyl, substituted or unsubstituted C3~C10 alkoxy, substituted or unsubstituted C5~C12 cycloalkyl, substituted or unsubstituted C5~C10 unsaturated hydrocarbyl, halogen or cyano, more preferably hydrogen, methyl, ethyl, n-propyl, isopropyl, n-butyl, isobutyl, tert-butyl, n-pentyl, isopentyl, n-hexyl, n-heptyl, hydroxymethyl, hydroxyethyl, 2-hydroxypropyl, 3-hydroxypropyl, 2-hydroxybutyl, 2-hydroxypentyl, fluoromethyl, difluoromethyl, trifluoromethyl, 1-fluoroethyl, 1-fluoropropyl, 1-fluoroisopropyl, 1-fluorobutyl, 1,1-difluoroethyl, 1,1-difluoropropyl, 1,1-difluorobutyl, methoxy, ethoxy, propoxy, butoxy, pentoxy, cyclopropyl, cyclobutyl, cyclopentyl, cyclohexyl, phenyl, naphthyl, anthracenyl, pyridyl, pyrrolyl, furyl, pyranyl, 1-methylvinyl, propenyl, butenyl, hydroxyl, fluorine, chlorine, bromine or iodine.

More specifically, the structure represented by formula (I) is specifically:

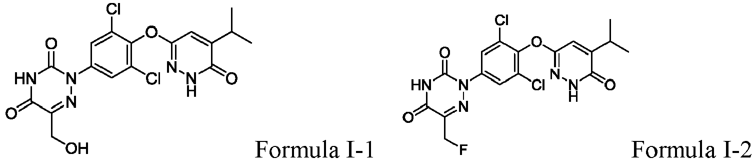

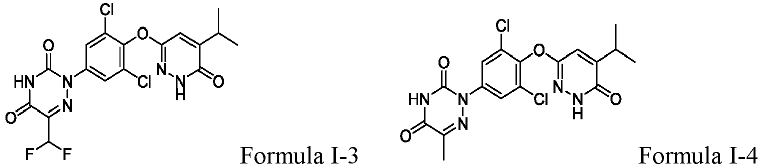

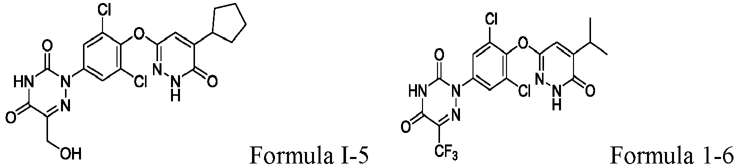

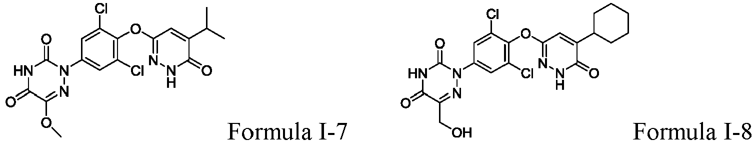

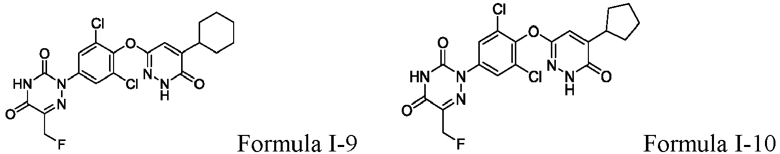

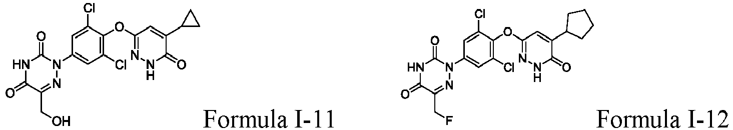

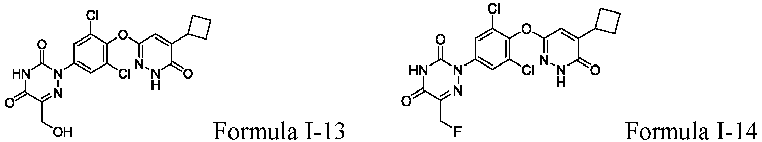

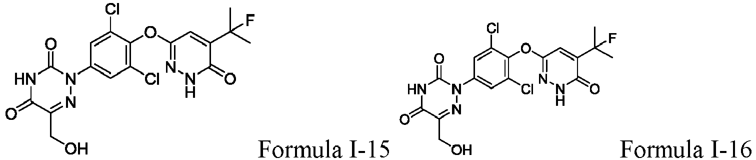

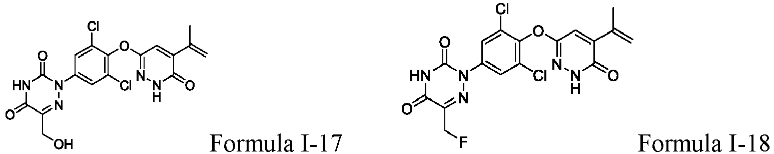

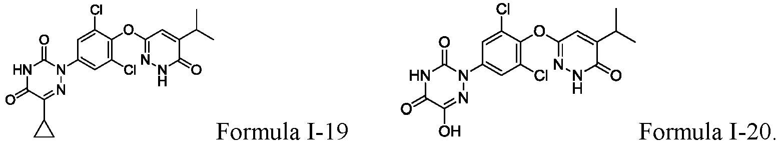

The present disclosure provides a method of preparing a 1,2,4-triazine-3,5-dione compound, comprising:
converting a compound of formula 1b into a compound of formula 1c, and then converting the compound of formula 1c into a 1,2,4-triazine-3,5-dione compound having a structure represented by formula (I), 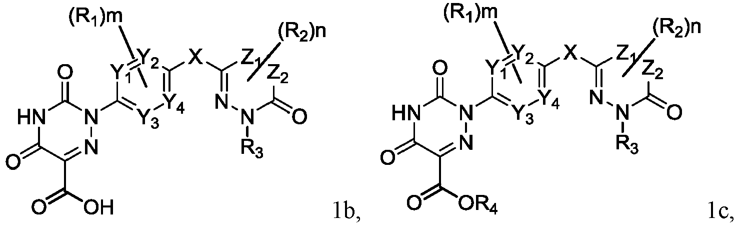 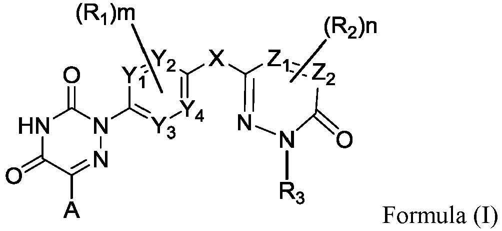
wherein, A is substituted or unsubstituted C1~C15 alkyl, substituted or unsubstituted C1~C15 alkoxy, substituted or unsubstituted C3~C18 cycloalkyl, substituted or unsubstituted C6~C20 aryl, substituted or unsubstituted C5~C20 heteroaryl, or substituted or unsubstituted C3~C18 heterocyclyl, hydroxyl or halogen;
X is substituted methylene, -O- or -S-,
Y₁, Y₂, Y₃, Y₄, Z₁ and Z₂ are independently selected from N and CR,
R₁, R₂, R₃ and R are independently selected from hydrogen, substituted or unsubstituted C1~C15 alkyl, substituted or unsubstituted C1~C15 alkoxy, substituted or unsubstituted C3~C18 cycloalkyl, and substituted or unsubstituted C2~C15 unsaturated hydrocarbyl, halogen or cyano;
m and n are each an integer of 0-3;
R₄ is C1~C6 alkyl.

According to the present disclosure, the present disclosure converts a compound of formula 1b into a compound of formula 1c. Specifically, in the present disclosure, compound 1b undergoes an esterification reaction with an alcohol under an acidic condition, wherein, an acid providing the acidic condition is selected from the group consisting of hydrochloric acid, sulfuric acid, glacial acetic acid, trifluoroacetic acid and a combination thereof; and the alcohol includes but is not limited to methanol and ethanol.

In the present disclosure, the present disclosure has no special requirements for the preparation method of compound 1b, which can be obtained by hydrolyzing compound 1a under an acidic condition. Specifically, an acid providing the acidic condition of the hydrolysis is selected from the group consisting of hydrochloric acid, sulfuric acid, glacial acetic acid, trifluoroacetic acid and a combination thereof, and the solvent of the reaction is N,N-dimethylformamide.

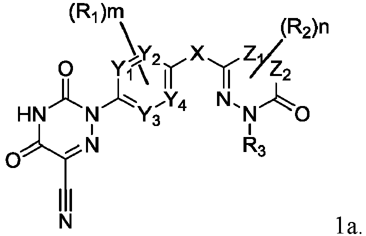

According to the present disclosure, the present disclosure also converts the compound of formula 1c into a 1,2,4-triazine-3,5-dione compound having the structure represented by formula (I). Specifically, in the present disclosure, compound 1c is reacted under the action of a reducing agent to obtain a compound having the structure represented by formula (I), wherein the reducing agent includes but is not limited to lithium aluminum hydride, sodium borohydride, lithium borohydride, DIBAL-H, vitride and the like. The reaction is carried out in an organic solvent including, but not limited to, methanol, ethanol, tetrahydrofuran, 1,4-dioxane, and the like.

Specifically, the reaction process is as follows:

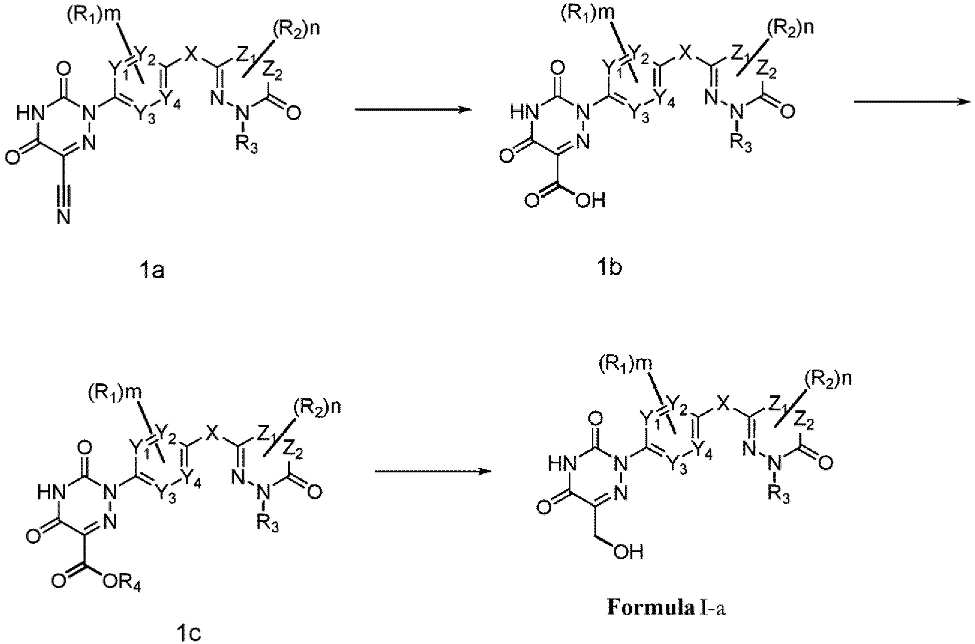

Wherein, the substitution of each group is the same as defined above.

Specifically, the present disclosure also subjects the compound of formula (I-a) to a fluorination reaction to obtain a compound of formula (I-b), wherein the reagent used in the fluorination reaction includes but is not limited to DAST, HF, n-Bu4NF, SF4, NaF, KF, AgF, HgF2, SbF3, etc. The specific reaction formula is as follows:

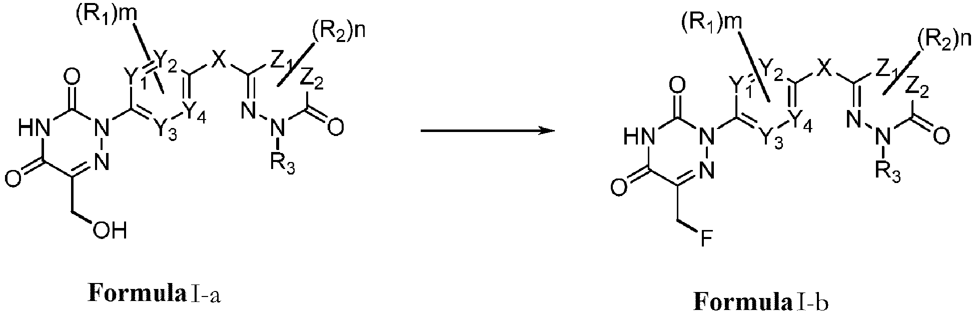

Each of R, R1, R2, R3, X, Y1, Y2, Y3, Y4, Z1, Z2, m and n has the meaning as described in the present disclosure.

Specifically, the present disclosure subjects the compound of formula (I-a) to oxidation followed by fluorination to obtain a compound of formula (I-c), wherein the oxidant for oxidation includes but is not limited to PCC, MnO₂, KMnO₄, IBX, PhI(OAc)₂, Dess-Martin reagent, etc.; and the oxidation reaction is carried out in an organic solvent including but not limited to dichloromethane, chloroform, carbon tetrachloride, DMF, tetrahydrofuran, and the like.

The specific reaction process is as follows:

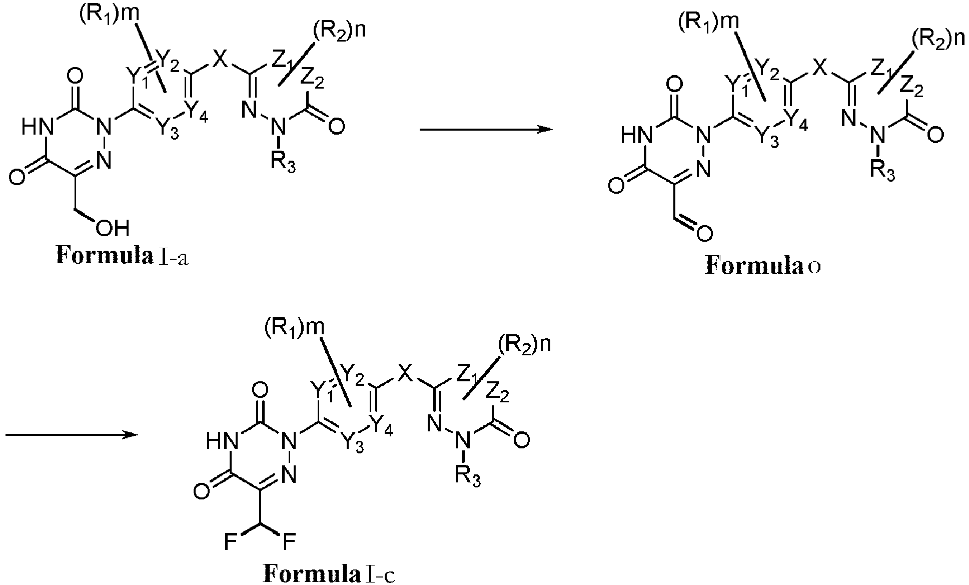

Each of R, R1, R2, R3, X, Y1, Y2, Y3, Y4, Z1, Z2, m and n has the meaning as described in the present disclosure.
m and n are each an integer of 0-3.

The present disclosure also provides a method of preparing a 1,2,4-triazine-3,5-dione compound and pharmaceutically acceptable salt thereof, comprising:
reacting compound 4d with compound 4e to obtain a 1,2,4-triazine-3,5-dione compound having a structure represented by formula (I), 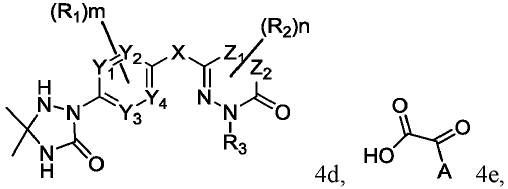 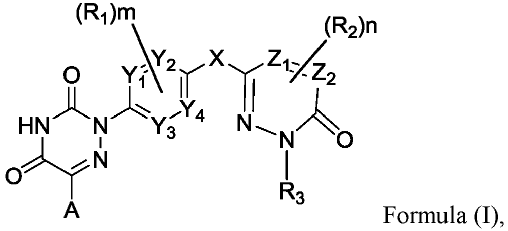
wherein, A is substituted or unsubstituted C1~C15 alkyl, substituted or unsubstituted C1~C15 alkoxy, substituted or unsubstituted C3~C18 cycloalkyl, substituted or unsubstituted C6~C20 aryl, substituted or unsubstituted C5~C20 heteroaryl, or substituted or unsubstituted C3~C18 heterocyclyl, hydroxyl or halogen;
X is substituted methylene, -O- or -S-,
Y₁, Y₂, Y₃, Y₄, Z₁ and Z₂ are independently selected from N and CR,
R₁, R₂, R₃ and R are independently selected from hydrogen, substituted or unsubstituted C1~C15 alkyl, substituted or unsubstituted C1~C15 alkoxy, substituted or unsubstituted C3~C18 cycloalkyl, and substituted or unsubstituted C2~C15 unsaturated hydrocarbyl, halogen or cyano; and
m and n are each an integer of 0-3.

According to the present disclosure, compound 4d is prepared by:
converting a compound of formula 4c into a compound of formula 4d, 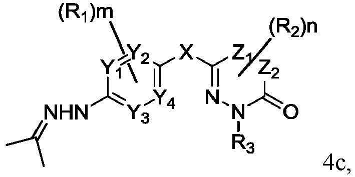
wherein X is substituted methylene, -O- or -S-,
Y₁, Y₂, Y₃, Y₄, Z₁ and Z₂ are independently selected from N and CR,
R₁, R₂, R₃ and R are independently selected from hydrogen, substituted or unsubstituted C1~C15 alkyl, substituted or unsubstituted C1~C15 alkoxy, substituted or unsubstituted C3~C18 cycloalkyl, and substituted or unsubstituted C2~C15 unsaturated hydrocarbyl, halogen or cyano; and
m and n are each an integer of 0-3.

In the present disclosure, compound 4c is obtained by condensing compound 4b with acetone under an acidic condition, and compound 4b is obtained by reacting compound 4a with sodium nitrite under an acidic condition.

Specifically, the reaction process is as follows:

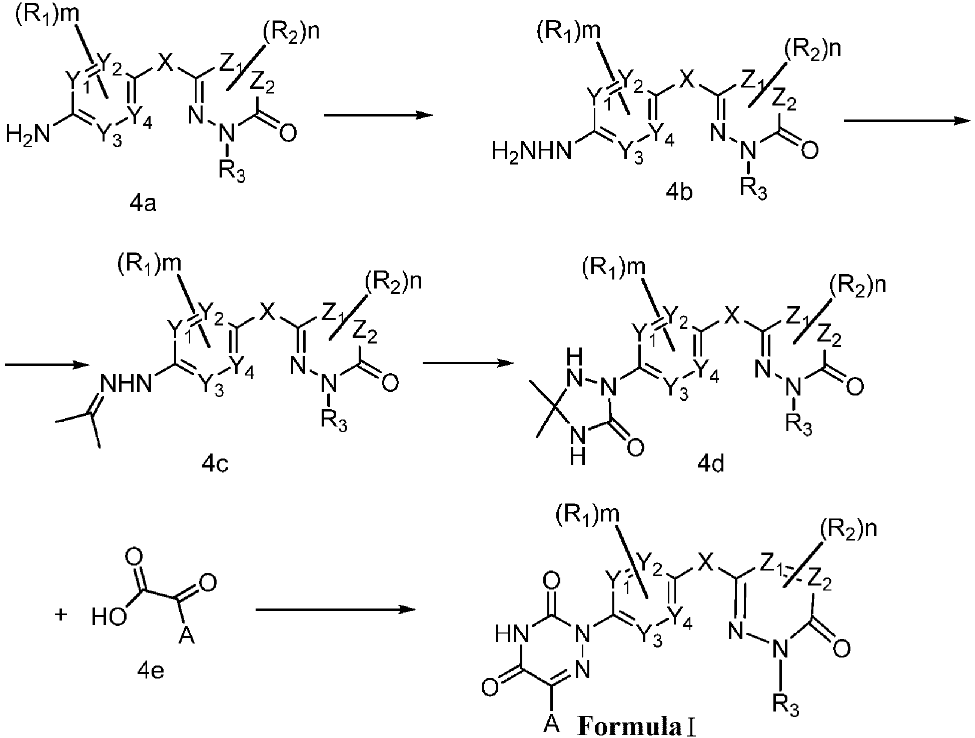

Wherein, compound 4a can be prepared by methods in literature. Compound 4b can be prepared by reacting compound 4a with sodium nitrite under an acidic condition to obtain the corresponding diazonium compound, which is then reacted with stannous chloride under an acidic condition. Compound 4c is obtained by condensing compound 4b with acetone under an acidic condition. Compound 4d is prepared by reacting compound 4c with potassium cyanate under an acidic condition. Compound formula (I) is obtained by condensing compound 4d with compound 4e under an acidic condition. The acidic condition includes but is not limited to, hydrochloric acid, sulfuric acid, formic acid, glacial acetic acid, trifluoroacetic acid, p-toluenesulfonic acid, methanesulfonic acid, and the like.

The present disclosure also provides use of the 1,2,4-triazine-3,5-dione compound and pharmaceutically acceptable salt thereof of the present disclosure in the manufacture of a THR agonist, and preferably, the THR agonist is a THRβ agonist; the compounds provided by the present disclosure can also be applied to diseases due to the influence of thyroid hormone receptors, including but not limited to obesity, diabetes, hypercholesterolemia, hyperlipidemia, hypertriglyceridemia hepatic steatosis, nonalcoholic fatty liver disease, nonalcoholic steatohepatitis, familial hypercholesterolemia, dyslipidemia, atherosclerosis, hypothyroidism, and thyroid cancer.

Groups represented by terms and symbols in this specification are explained below.

"Alkyl" when used as a group or part of a group is meant to include straight or branched C1-C20 aliphatic hydrocarbon groups, preferably C1-C10 alkyl, more preferably C1-C6 alkyl groups, particularly preferably C1-C4 alkyl. Examples of alkyl groups include, but are not limited to, methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, and the like. Alkyl groups may be substituted or unsubstituted.

"Alkylene" is a divalent alkyl group, preferably C1-C10 alkylene, more preferably C1-C6 alkylene, particularly preferably C1-C4 alkylene. Examples of alkylene groups include, but are not limited to, methylene, ethylene, 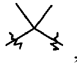 , n-propylene, and the like. Alkylene groups may be substituted or unsubstituted.

"Alkenyl" refers to an alkyl group as defined above consisting of at least two carbon atoms and at least one carbon-carbon double bond, and representative examples include, but are not limited to, ethenyl, 1-propenyl, 2-propenyl, 1-, 2- or 3-butenyl, and the like, preferably C2-C4 alkylene. Alkenyl groups may be optionally substituted or unsubstituted.

"Alkynyl" when used as a group or part of a group refers to an aliphatic hydrocarbon group containing a carbon-carbon triple bond, which may be straight or branched, preferably C2-C10 alkynyl, more preferably C2-C6 alkynyl, and most preferably C2-C4 alkynyl. Examples of alkynyl groups include, but are not limited to, ethynyl, 1-propynyl, 2-propynyl, 1-, 2- or 3-butynyl, and the like. Alkynyl groups may be substituted or unsubstituted.

"Cycloalkyl" means a saturated or partially saturated monocyclic, fused, bridged or spiro carbocyclic ring, preferably C3-C12 cycloalkyl, more preferably C3-C8 cycloalkyl, and most preferably C3-C6 cycloalkyl. Examples of monocyclic cycloalkyl groups include, but are not limited to, cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like, preferably cyclopropyl and cyclohexenyl.

"Cycloalkylene" is a divalent cycloalkyl group, preferably C3-C12 cycloalkylene, more preferably C3-C8 cycloalkylene, and most preferably C3-C6 cycloalkylene. Examples of alkylene groups include, but are not limited to, cyclopropylene, cyclobutylene, cyclopentylene, and the like. Cycloalkylene groups may be substituted or unsubstituted.

"Cyclopropylidene" means

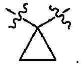

"Cyclobutylene" means

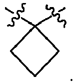

"Spirocycloalkyl" refers to a 5- to 18-membered, preferably 6- to 14-membered, more preferably 7- to 10-membered polycyclic group containing two or more cyclic structures with the monocyclic rings sharing one carbon atom (called a spiro atom) with each other, in which the rings may contain one or more double bonds, but none of the rings has an aromatic system with fully conjugated π electrons. According to the number of spiro atoms shared between the rings, spirocycloalkyl groups are divided into mono-spiro, double-spiro or poly-spirocycloalkyl groups, preferably mono-spiro and double-spirocycloalkyl groups, preferably 4-membered/5-membered, 4-membered/6-membered, 5-membered/5-membered or 5-membered/6-membered. Non-limiting examples of "spirocycloalkyl" include, but are not limited to, spiro[4.5]decyl, spiro[4.4]nonyl, spiro[3.5]nonyl, and spiro[2.4]heptyl.

"Fused cycloalkyl" refers to a 5- to 18-membered, preferably 6- to 12-membered, more preferably 7- to 10-membered all-carbon polycyclic group containing two or more cyclic structures that share a pair of carbon atoms with each other, in which one or more rings may contain one or more double bonds, but none of the rings has an aromatic system with fully conjugated π electrons. According to the number of formed rings, it can be divided into bicyclic, tricyclic, pyridone or polycyclic fused cycloalkyl, preferably bicyclic or tricyclic, more preferably 5-membered/5-membered or 5-membered/6-membered bicycloalkyl. Non-limiting examples of "fused cycloalkyl" include, but are not limited to: bicyclo[3.1.0]hexyl, bicyclo[3.2.0]hept-1-enyl, bicyclo[3.2.0]heptyl, decahydronaphthyl and tetradecahydrophenanthrenyl.

"Bridged cycloalkyl" refers to a 5- to 18-membered, preferably 6- to 14-membered, more preferably 7- to 10-membered all-carbon polycyclic group containing two or more cyclic structures that share two carbon atoms that are not directly attached to each other, in which one or more rings may contain one or more double bonds, but none of the rings has an aromatic system with fully conjugated π electrons. According to the number of constituent rings, it can be divided into bicyclic, tricyclic, pyridone or polycyclic bridged cycloalkyl, preferably bicyclic, tricyclic or pyridone, more preferably bicyclic or tricyclic. Non-limiting examples of "bridged cycloalkyl" include, but are not limited to: (1s,4s)-bicyclo[2.2.1]heptyl, bicyclo[3.2.1]octyl, (1s,5s)-bicyclo[3.3.1]nonyl, bicyclo[2.2.2]octyl, and (1r,5r)-bicyclo[3.3.2]decyl.

The cycloalkyl ring can be fused to an aryl, heteroaryl or heterocyclyl ring, in which the ring attached to the parent structure is cycloalkyl, and non-limiting examples include indanyl, tetrahydronaphthyl, benzocycloheptyl, and the like. Cycloalkyl groups may be optionally substituted or unsubstituted.

"Heterocyclyl," "heterocycle," or "heterocyclic" are used interchangeably herein to refer to a non-aromatic heterocyclyl in which one or more of the ring-forming atoms is a heteroatom, such as oxygen, nitrogen, sulfur atoms, etc., including monocyclic, fused, bridged and spiro rings. It preferably has a 5- to 7-membered monocyclic ring or a 7- to 10-membered bi- or tricyclic ring, which may contain 1, 2 or 3 atoms selected from nitrogen, oxygen and/or sulfur. Examples of "heterocyclyl" include, but are not limited to, morpholinyl, oxetanyl, thiomorpholinyl, tetrahydropyranyl, 1,1-dioxo-thiomorpholinyl, piperidinyl, 2-oxo-piperidinyl, pyrrolidinyl, 2-oxo-pyrrolidinyl, piperazin-2-one, 8-oxa-3-aza-bicyclo[3.2.1]octyl and piperazinyl. Heterocyclyl groups may be substituted or unsubstituted.

"Spiroheterocyclyl" refers to a 5- to 18-membered, preferably 6- to 14-membered, more preferably 7- to 10-membered polycyclic group containing two or more cyclic structures with the monocyclic rings sharing one atom with each other, in which the ring contains one or more double bonds, but none of the rings has an aromatic system with fully conjugated π electrons, where one or more ring atoms are selected from nitrogen, oxygen, or a heteroatom of S(O)q (where q is selected from 0, 1, or 2), and the rest ring atoms are carbon. According to the number of spiro atoms shared between rings, spirocycloalkyl groups are divided into mono-spiroheterocyclyl, bis-spiroheterocyclyl or poly-spiroheterocyclyl, preferably mono-spiroheterocyclyl and bis-spiroheterocyclyl, more preferably 4-membered/4-membered, 4-membered/5-membered, 4-membered/6-membered, 5-membered/5-membered or 5-membered/6-membered mono-spiroheterocyclyl. Non-limiting examples of "spiroheterocyclyl" include, but are not limited to: 1,7-dioxaspiro[4.5]decyl, 2-oxa-7-azaspiro[4.4]nonyl, 7-oxaspiro[3.5]nonyl and 5-oxaspiro[2.4]heptyl.

"Fused heterocyclyl" refers to an all-carbon polycyclic group containing two or more ring structures that share a pair of atoms with each other, in which one or more rings may contain one or more double bonds, but none of the rings has an aromatic system with fully conjugated π electrons, where one or more ring atoms are selected from nitrogen, oxygen, or a heteroatom of S(O)q (where q is selected from 0, 1, or 2), and the rest ring atoms are carbon. It is preferably 6-to 14-membered, more preferably 7- to 10-membered. According to the number of constituent rings, it can be divided into bicyclic, tricyclic, pyridone or polycyclic fused heterocyclic groups, preferably bicyclic or tricyclic, more preferably 5-membered/5-membered or 5-membered/6-membered bicyclic fused heterocyclic groups. Non-limiting examples of "fused heterocyclyl" include, but are not limited to: octahydropyrrolo[3,4-c]pyrrolyl, octahydro-1H-isoindolyl, 3-azabicyclo[3.1. 0]hexyl, and octahydrobenzo[b][1,4]dioxine.

"Bridged heterocyclyl" refers to a 5- to 18-membered, preferably 5- to 14-membered polycyclic group containing two or more cyclic structures that share two atoms which are not directly attached to each other, in which one or more rings may contain one or more double bonds, but none of the rings have an aromatic system with fully conjugated π electrons, where one or more ring atoms are selected from nitrogen, oxygen, or a heteroatom of S(O)q (where q is selected from 0, 1, or 2), and the rest ring atoms are carbon. It is preferably 6- to 14-membered, more preferably 7- to 10-membered. According to the number of constituent rings, it can be divided into bicyclic, tricyclic, pyridone or polycyclic bridged heterocyclic groups, preferably bicyclic, tricyclic or pyridone, more preferably bicyclic or tricyclic. Non-limiting examples of "fused heterocyclyl" include, but are not limited to: 2-azabicyclo[2.2.1]heptyl, 2-azabicyclo[2.2.2]octyl, and 2-azabicyclo[3.3.2]decyl. The heterocyclyl ring can be fused to an aryl, heteroaryl or cycloalkyl ring, in which the ring attached to the parent structure is heterocyclyl. Heterocyclyl groups may be optionally substituted or unsubstituted.

"Heterocyclylene" refers to a divalent heterocyclyl group, preferably a 5- to 7-membered monocyclic heterocyclylene or a 7- to 10-membered bicyclic heterocyclylene or a tricyclic heterocyclylene, which may contain 1, 2 or 3 atoms selected from nitrogen, oxygen and/or sulfur. Heterocyclylene groups may be substituted or unsubstituted.

"Aryl" refers to a carbocyclic aromatic system containing one or two rings, wherein the rings may be attached together in a fused fashion. The term "aryl" includes aromatic groups such as phenyl, naphthyl, tetrahydronaphthyl. Preferred aryl is C6-C10 aryl, more preferred aryl is phenyl and naphthyl, and most preferred is phenyl. Aryl groups may be substituted or unsubstituted. The "aryl" can be fused with a heteroaryl, a heterocyclyl or a cycloalkyl, in which the ring attached to the parent structure is an aryl ring, and non-limiting examples include but are not limited to:

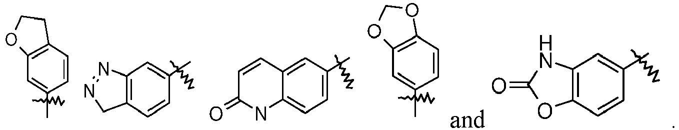

"Heteroaryl" refers to an aromatic 5- to 6-membered monocyclic or 9- to 10-membered bicyclic ring, which may contain 1 to 4 atoms selected from nitrogen, oxygen, and/or sulfur. Examples of "heteroaryl" include, but are not limited to, furyl, pyridyl, 2-oxo-1,2-dihydropyridyl, pyridazinyl, pyrimidinyl, pyrazinyl, thienyl, isoxazolyl, oxazolyl, oxadiazolyl, imidazolyl, pyrrolyl, pyrazolyl, triazolyl, tetrazolyl, thiazolyl, isothiazolyl, 1,2,3-thiadiazolyl, benzodioxolyl, benzimidazolyl, indolyl, isoindolyl, 1,3-dioxo-isoindolyl, quinolinyl, indazolyl, benzisothiazolyl, benzoxazolyl and benzisoxazolyl. Heteroaryl groups may be substituted or unsubstituted. The heteroaryl ring can be fused to an aryl, heterocyclyl or cycloalkyl ring, in which the ring attached to the parent structure is a heteroaryl ring, and non-limiting examples include but are not limited to:

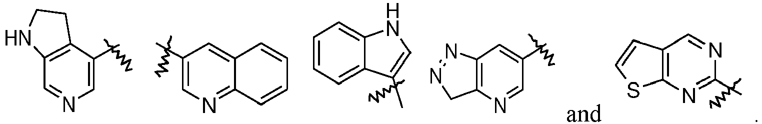

"Alkoxy" refers to a (alkyl-O-) group, wherein alkyl is as defined herein. C1-C6 alkoxy is preferred, and C1-C4 alkoxy is particularly preferred. Examples include, but are not limited to, methoxy, ethoxy, n-propoxy, isopropoxy, n-butoxy, isobutoxy, tert-butoxy, and the like.

"Hydroxy" refers to the -OH group.

"Halogen" refers to fluorine, chlorine, bromine and iodine, preferably chlorine, bromine and iodine.

"Amino" refers to -NH2.

"Cyano" refers to -CN.

"Nitro" refers to -NO2.

"Benzyl" refers to -CH2-phenyl.

"Carboxyl" refers to -C(O)OH.

"Carboxylate" refers to -C(O)O(alkyl) or (cycloalkyl), wherein alkyl and cycloalkyl are as defined above.

"DMSO" refers to dimethyl sulfoxide.

"Sulfhydryl" refers to -SH.

"Substituted" means that one or more hydrogen atoms, preferably up to 5, more preferably 1 to 3 hydrogen atoms in a group, independently of each other, are replaced by the corresponding number of substituents. It goes without saying that the substituents are only in their possible chemical positions, and those skilled in the art can determine (either experimentally or theoretically) possible or impossible substitutions without undue effort. For example, amino or hydroxyl with free hydrogens may be unstable when bound to carbon atoms with unsaturated bonds such as ethylenic bonds.

"Substitution" or "substituted" mentioned in this specification, unless otherwise specified, means that the group may be substituted by one or more groups selected from the group consisting of: alkyl, alkenyl, alkynyl, alkoxy, alkylthio, alkylamino, halogen, sulfhydryl, hydroxyl, nitro, cyano, cycloalkyl, heterocyclyl, aryl, heteroaryl, cycloalkoxy, heterocycloalkoxy, cycloalkylthio, heterocycloalkylthio, amino, haloalkyl, hydroxyalkyl, carboxyl, carboxylate, =O, -OR11, -SR11, -NR9R10, -C(O)NR9R10, -C(O)R11, -OC(O)R11, -S(O)nNR9R10, -C(O)OR11 or -NR9C(O)R10, wherein n is 0, 1 or 2;
"Pharmaceutically acceptable salt" refers to certain salts of the above-mentioned compounds that retain their original biological activity and are suitable for medicinal use. The pharmaceutically acceptable salt of the compound represented by formula (I) may be metal salts and amine salts formed with suitable acids. Metal salts are preferably alkali metal salts and alkaline earth metal salts. Suitable acids include inorganic and organic acids, such as acetic acid, benzenesulfonic acid, benzoic acid, camphorsulfonic acid, citric acid, ethanesulfonic acid, fumaric acid, gluconic acid, glutamic acid, hydrobromic acid, hydrochloric acid, isethionic acid, lactic acid, malic acid, maleic acid, mandelic acid, methanesulfonic acid, nitric acid, phosphoric acid, succinic acid, sulfuric acid, tartaric acid, p-toluenesulfonic acid, and the like. Particularly preferred are hydrochloric acid, hydrobromic acid, phosphoric acid and sulfuric acid, and most preferred is hydrochloric acid.

"Pharmaceutical composition" means a mixture containing one or more of the compounds (including their pharmaceutically acceptable salts or in the form of stereoisomers, tautomers or prodrugs, etc.) described herein and optionally additional pharmaceutically active ingredients, which may contain additional optional components such as pharmaceutically acceptable carriers and/or excipients. The purpose of the pharmaceutical composition is to facilitate the administration to the organism, which contributes to the absorption of the active ingredient and then exerts the biological activity.

As used herein, the term "more" includes two or more, such as two, three, four, etc.

1H NMR spectrum was obtained with a Bruker instrument (400 MHz) with chemical shifts expressed in ppm. A tetramethylsilane internal standard (0.00 ppm) was used. 1H NMR notation: s=singlet, d=doublet, t=triplet, q=quartet, m=multiplet, br=broadened, dd=doublet of doublet, dt= doublet of triplet. When coupling constants are provided, they are in Hz.

Mass spectrometry was performed using a Shimadzu LCMS-2020 liquid mass spectrometer, and the ionization method may be ESI or APCI.

Preparation and purification were performed using a Shimadzu LC-20AP preparative high performance liquid chromatograph.

Flash column chromatography was performed using a Biotage Isolera^{™} Prime flash preparative chromatograph.

Microwave reaction was performed using an Anton Paar Monowave 400 microwave reactor.

The thin layer chromatography silica gel plate was performed by using Yantai Huanghai HSGF254 or Qingdao GF254 silica gel plate. The size of the silica gel plate used for thin layer chromatography (TLC) was 0.15 mm ~ 0.2 mm, and the size used for the separation and purification of products by TLC was 0.4 mm ~0.5 mm.

For column chromatography, Yantai Huanghai silica gel 200~300 mesh silica gel was usually used as the carrier.

In the following examples, unless otherwise indicated, all temperatures are in degrees Celsius, and unless otherwise indicated, various starting materials and reagents are obtained from commercial sources or synthesized according to known methods. Commercially available starting materials and reagents are used without further purification. Unless otherwise specified, commercial manufacturers include but are not limited to Aldrich Chemical Company, ABCR GmbH & Co.KG, Acros Organics, Guangzan Chemical Technology Co., Ltd. and Jingyan Chemical Technology Co., Ltd., etc.
D3OD: deuterated methanol
CDC13: deuterated chloroform
DMSO-d6: deuterated dimethyl sulfoxide

Argon atmosphere means that the reaction flask is connected to an argon balloon with a volume of about 1 L.

Nitrogen atmosphere means that the reaction flask is connected to a nitrogen balloon with a volume of about 1 L.

Unless otherwise specified in the examples, the solution in the reaction refers to an aqueous solution.

The compound is purified by silica gel column chromatography and thin layer chromatography, in which the eluent system is selected from: A: petroleum ether and ethyl acetate B: dichloromethane and methanol; C: dichloromethane: ethyl acetate; the volume ratio of the solvents varies according to the polarity of the compound, and can also be adjusted by adding a small amount of acidic or basic reagents, such as acetic acid or triethylamine, etc.

The present disclosure will be described clearly and completely in conjunction with the technical embodiments of the examples of the present disclosure. Apparently, the described examples are only a part of the examples of the present disclosure, rather than all the examples. Based on the examples of the present disclosure, all other examples obtained by those of ordinary skill in the art without creative efforts shall fall within the protection scope of the present disclosure.

### Example 1

### 2-(3,5-Dichloro-4-((5-isopropyl-6-oxy-1,6-dihydropyridazin-3-yl)oxy)phenyl)-(hydroxymethyl)-1,2,4-triazine-3,5(2H,4H)-dione

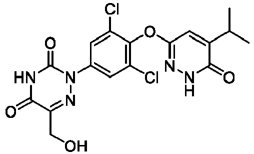

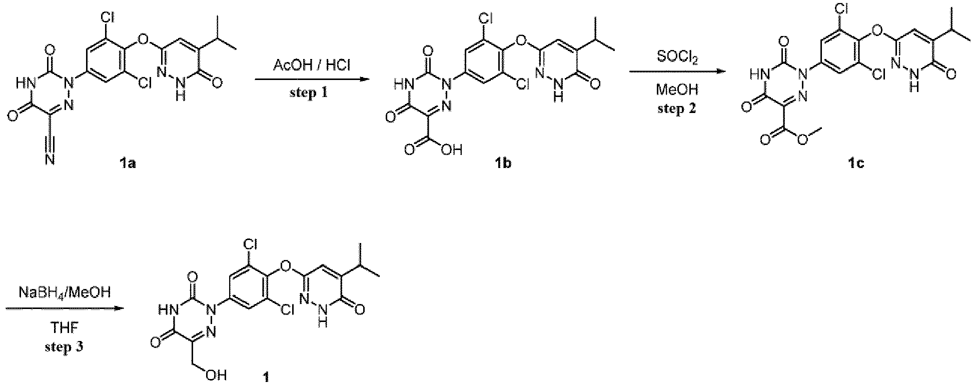

### Step 1

### 2-(3,5-Dichloro-4-((5-isopropyl-6-oxy-1,6-dihydropyridazin-3-yl)oxy)phenyl)-3,5-dioxo-2,3,4,5-tetrahydro-1,2,4-triazine-6-carboxylic acid

2-(3,5-Dichloro-4-((5-isopropyl-6-oxy-1,6-dihydropyridazin-3-yl)oxy)phenyl)-3,5-diox o-2,3,4,5-tetrahydro-1,2,4-triazine-6-cyano **1a** (300 mg, 0.69 mmol, see WO2014043706A1 for the synthesis method) was dissolved in 10 mL of glacial acetic acid, added with concentrated hydrochloric acid (2 mL), and heated to 120°C for 5 h of reaction. After the completion of the reaction, the reaction solution was cooled to room temperature, added with 20 mL of water to dilute, stirred, filtered, and dried to obtain 2-(3,5-dichloro-4-((5-isopropyl-6-oxy-1,6-dihydropyridazin-3-yl)oxy)phenyl)-3,5-dioxo-2,3,4,5-t etrahydro-1,2,4-triazine-6-carboxylic acid **1b** (260 mg, pale yellow solid), yield: 84%.
¹H NMR (400 MHz, DMSO-*d₆*) δ 12.71 (s, 1H), 12.21 (s, 1H), 7.82 (s, 2H), 7.45 (s, 1H), 3.21 (m, 1H), 1.19 (d, *J* = 6.4 Hz, 6H). MS m/z (ESI): 453.8 [M+1]⁺.

### Step 2

### Methyl 2-(3,5-dichloro-4-((5-isopropyl-6-oxy-1,6-dihydropyridazin-3-yl)oxy)phenyl)-3,5-dioxo-2,3,4,5-t etrahydro-1,2,4-triazine-6-carboxylate

2-(3,5-Dichloro-4-((5-isopropyl-6-oxy-1,6-dihydropyridazin-3-yl)oxy)phenyl)-3,5-diox y-2,3,4,5-tetrahydro-1,2,4-triazine-6-carboxylic acid **1b** (200 mg, 0.44 mmol) was dissolved in 20 mL methanol, and thionyl chloride (2 mL) was added slowly under ice bath. The reaction solution was heated to 100°C for 5 h of reaction. After the completion of the reaction, the reaction solution was concentrated under reduced pressure, and the residual solid was slurried with petroleum ether: ethyl acetate (10:1), filtered, and dried to obtain methyl 2-(3,5-dichloro-4-((5-isopropyl-6-oxy-1,6-dihydropyridazin-3-yl)oxy)phenyl)-3,5-dioxo-2,3,4,5-t etrahydro-1,2,4-triazine-6-carboxylate **1c** (200 mg, pale yellow solid), yield: 97%. MS m/z (ESI): 466.2 [M-1]⁻.

### Step 3

### 2-(3,5-Dichloro-4-((5-isopropyl-6-oxy-1,6-dihydropyridazin-3-yl)oxy)phenyl)-(hydroxymethyl)-1,2,4-triazine-3,5(2H,4H)-dione

Methyl 2-(3,5-dichloro-4-((5-isopropyl-6-oxy-1,6-dihydropyridazin-3-yl)oxy)phenyl)-3,5-dioxo-2,3,4,5-t etrahydro-1,2,4-triazine-6-carboxylate **1c** (200 mg, 0.43 mmol) was dissolved in 20 mL of tetrahydrofuran, added with sodium borohydride (82 mg, 2.15 mmol) at room temperature, and then slowly added with methanol dropwise. After the completion of the reaction, the reaction solution was added with 20 mL of water, adjusted to pH = 6 with 3M dilute hydrochloric acid, and extracted with ethyl acetate (30 mL × 3). The organic phases were combined, washed with saturated sodium chloride solution (100 mL), dried over anhydrous sodium sulfate, filtered, and concentrated under reduced pressure. The residue was purified by silica gel column chromatography (eluent: petroleum ether: ethyl acetate=5:1~0:1) to obtain 2-(3,5-dichloro-4)-((5-isopropyl-6-oxy-1,6-dihydropyridazin-3-yl)oxy)phenyl)-(hydroxymethyl)-1,2,4triazine-3,5(2H,4H)-dione **1** (150 mg, white solid), yield: 79%.
MS m/z(ESI): 438.2 [M-1]⁻. ¹H NMR (400 MHz, DMSO-*d₆*) δ 12.49 (s, 1H), 12.25 (s, 1H), 7.86 (s, 2H), 7.45 (s, 1H), 5.33 (t, *J* = 6.4 Hz, 1H), 4.40 (d, *J* = 6.0 Hz, 2H), 3.21 (m, 1H), 1.19 (d,*J* = 6.4 Hz, 6H).

### Example 2

### 2-(3,5-Dichloro-4-((5-isopropyl-6-oxy-1,6-dihydropyridin-3-yl)oxy)phenyl)-(fluoromethyl)-1,2,4 -triazine-3,5(2H,4H)-dione

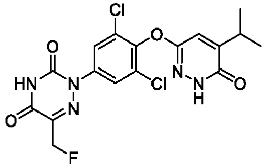

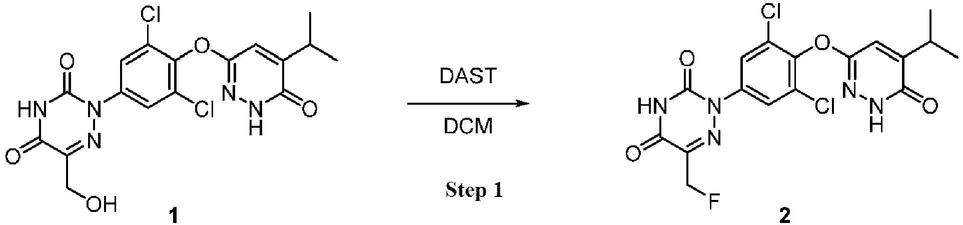

### Step 1

### 2-(3,5-Dichloro-4-((5-isopropyl-6-oxy-1,6-dihydropyridin-3-yl)oxy)phenyl)-(fluoromethyl)-1,2,4 -triazine-3,5(2H,4H)-dione

Methyl 2-(3,5-dichloro-4-((5-isopropyl-6-oxy-1,6-dihydropyridazin-3-yl)oxy)phenyl)-(hydroxymethyl)-1 ,2,4-triazine-3,5(2H,4H)-dione **1** (100 mg, 0.23 mmol) was dissolved in 20 mL of dichloromethane and added with DAST (5 drops) under ice bath, and the reaction solution was reacted at this temperature for 30 min. After the completion of the reaction, the reaction solution was added to 20 mL of ice water and extracted with dichloromethane (20 mL × 3). The organic phases were combined, washed with saturated sodium chloride solution (100 mL), dried over anhydrous sodium sulfate, filtered, and concentrated under reduced pressure. The residue was purified by preparative chromatography to obtain 2-(3,5-dichloro-4-((5-isopropyl-6-oxy-1,6-dihydropyridin-3-yl)oxy)phenyl)-(fluoromethyl)-1,2,4-triazine-3,5(2H,4H)-dione **2** (45 mg, white solid), yield: 45%. MS m/z (ESI): 442.2 [M+1]⁺. ¹H NMR (400 MHz, DMSO-*d₆*) δ 12.48 (s, 1H), 12.21 (s, 1H), 7.81 (s, 2H), 7.43 (s, 1H), 5.29 (d, *J* = 48.0 Hz, 1H), 3.21 (m, 1H), 1.19 (d, *J* = 6.4 Hz, 6H). ¹⁹F NMR (400 MHz, DMSO-*d₆*) δ -219.9 ppm.

### Example 3

### 2-(3,5-Dichloro-4-((5-isopropyl-6-oxy-1,6-dihydropyridazin-3-yl)oxy)phenyl)--(difluoromethyl)-1,2,4-triazine-3,5(2H,4H)-dione

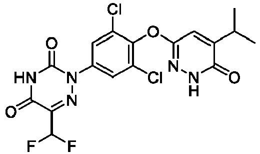

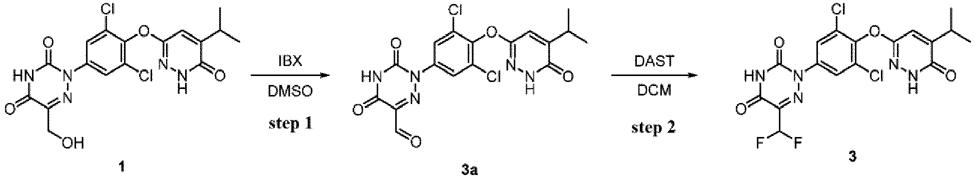

### Step 1

### 2-(3,5-Dichloro-4-((5-isopropyl-6-oxy-1,6-dihydropyridin-3-yl)oxy)phenyl)-3,5-dioxo-2,3,4,5-tet rahydro-1,2,4-triazine-6-carbaldehyde

Methyl 2-(3,5-dichloro-4-((5-isopropyl-6-oxy-1,6-dihydropyridazin-3-yl)oxy)phenyl)-(hydroxymethyl)-1 ,2,4-triazine-3,5(2H,4H)-dione **1** (100 mg, 0.23 mmol) was dissolved in 20 mL of dichloromethane and added with 2-iodoxybenzoic acid (129 mg, 0.46 mmol), and the reaction solution was heated to 50°C for 30 min. After the completion of the reaction, the reaction solution was added to 20 mL of ice water and extracted with ethyl acetate (50 mL × 3). The organic phases were combined, washed with saturated sodium chloride solution (100 mL), dried over anhydrous sodium sulfate, filtered, and concentrated under reduced pressure. The residue was purified by preparative chromatography to obtain 2-(3,5-dichloro-4-((5-isopropyl-6-oxy-1,6-dihydropyridin-3-yl)oxy)phenyl)-3,5-dioxo-2,3,4,5-tet rahydro-1,2,4-triazine-6-carbaldehyde **3a** (65 mg, white solid), yield: 65%. MS m/z (ESI): 438.2 [M+1]⁺.

### Step 2

### 2-(3,5-Dichloro-4-((5-isopropyl-6-oxy-1,6-dihydropyridazin-3-yl)oxy)phenyl)--(difluoromethyl)-1,2,4-triazine-3,5(2H,4H)-dione

2-(3,5-Dichloro-4-((5-isopropyl-6-oxy-1,6-dihydropyridin-3-yl)oxy)phenyl)-3,5-dioxo -2,3,4,5-tetrahydro-1,2,4-triazine-6-carbaldehyde **3a** (60 mg, 0.14 mmol) was dissolved in 20 mL of dichloromethane and added with DAST (1 mL) under ice bath, and the reaction solution was warmed to room temperature for 10 min. After the completion of the reaction, the reaction solution was added to 20 mL of ice water and extracted with dichloromethane (20 mL × 3). The organic phases were combined, washed with saturated sodium chloride solution (100 mL), dried over anhydrous sodium sulfate, filtered, and concentrated under reduced pressure. The residue was purified by preparative chromatography to obtain 2-(3,5-dichloro-4-((5-isopropyl-6-oxy-1,6-dihydropyridazin-3-yl)oxy)phenyl)-(difluoromethyl)-1 ,2,4-triazine-3,5(2H,4H)-dione **3** (40 mg, white solid), yield: 62 %.
¹H NMR (400 MHz, DMSO-*d₆*) δ 12.85 (s, 1H), 12.22 (s, 1H), 7.81 (s, 2H), 7.45 (s, 1H), 6.90 (t, *J* = 5.2 Hz, 1H), 3.21 (m, 1H), 1.19 (d, *J* = 6.4 Hz, 6H). ¹⁹F NMR (400 MHz, DMSO-*d₆*) δ -122.1 ppm. MS m/z (ESI): 459.9 [M+1]⁺.

### Example 4

### 2-(3,5-Dichloro-4-((5-isopropyl-6-oxy-1,6-dihydropyridazin-3-yl)oxy)phenyl)-6-methyl-1,2,4-tri azine-3, 5 (2H,4H)-dione

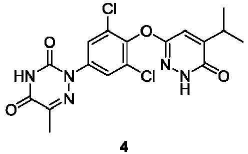

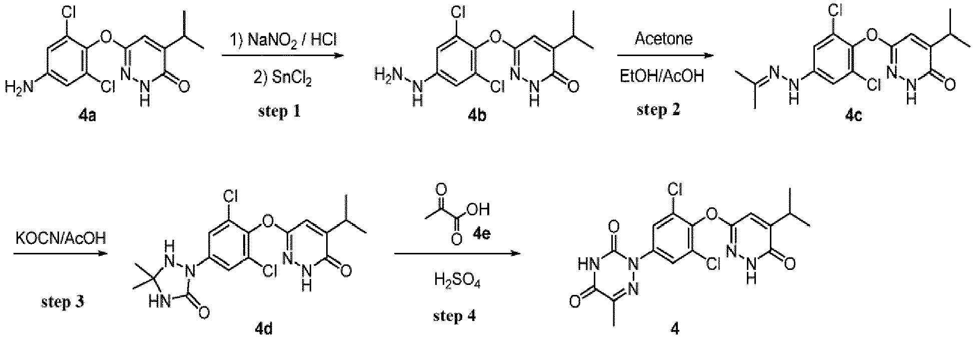

### Step 1

### 6-(2,6-Dichloro-4-hydrazinophenoxy)-4-isopropylpyridazin-3(2H)-one

6-(-4-Amino-2,6-dichlorophenoxy)-4-isopropylpyridazin-3(2H)-one **4a** (1.0 g, 3.19 mmol, see WO2014043706A1 for the synthesis method) was dissolved in 20 mL of 6M dilute hydrochloric acid and added with sodium nitrite (440 mg, 6.38 mmol) under ice bath, and the reaction solution was reacted at this temperature for 1 h. Then a solution of stannous chloride (1.21 g, 6.38 mmol) dissolved in 2 mL of hydrochloric acid solution was slowly added and to continue to react for 3 h. After the completion of the reaction, the reaction solution was added to 50 mL of ice water and extracted with ethyl acetate (50 mL × 3). The organic phases were combined, washed with saturated sodium chloride solution (200 mL), dried over anhydrous sodium sulfate, filtered, and concentrated under reduced pressure to obtain the crude compound 6-(2,6-dichloro-4-hydrazinophenoxy)-4-isopropylpyridazin-3(2H)-one **4b** (800 mg, yellow solid), yield: 79%. MS m/z (ESI): 329.1 [M+1]⁺.

### Step 2

### 6-(2,6-Dichloro-4-(2-(propyl-2-ethanediene)hydrazine)phenoxy)-4-isopropylpyridazin-3(2H)-one

6-(2,6-Dichloro-4-hydrazinophenoxy)-4-isopropylpyridazin-3(2H)-one **4b** (800 mg, 2.44 mmol) was dissolved in 50 mL of ethanol and added with acetone (5 mL) followed by acetic acid (1 mL), and the reaction solution was heated to 100°C and refluxed for 3 h. After the completion of the reaction, the reaction solution was concentrated under reduced pressure to obtain the crude compound 6-(2,6-dichloro-4-(2-(propyl-2-ethylenediene)hydrazino)phenoxy)-4-isopropylpyridazin-3(2H)-o ne **4c** (850 mg, brown oil), yield: 94%.

### Step 3

### 6-(2,6-Dichloro-4-(3,3-dimethyl-5-oxy-1,2,4-triazolidin-1-yl)phenoxy)-4-isopropylpyridazin-3(2 H)-one

6-(2,6-Dichloro-4-(2-(propyl-2-ethanediene)hydrazine)phenoxy)-4-isopropylpyridazin-3(2H)-one **4c** (850 mg, 2.3 mmol) was dissolved in 50 mL of a solution of acetic acid and water (5:1) and added with potassium cyanate, and the reaction was carried out at room temperature for 3 h. After the completion of the reaction, the reaction solution was added with 50 mL of water, stirred and filtered, and the filter cake was dried to obtain the crude compound 6-(2,6-dichloro-4-(3,3-dimethyl-5-oxo-1,2,4-triazolidin-1-yl)phenoxy)-4-isopropylpyridazin-3(2 H)-one **4d** (500 mg, yellow solid), yield: 53%.
¹H NMR (400 MHz, DMSO-*d₆*) δ 12.18 (s, 1H), 7.92 (s, 1H), 7.73 (s, 2H), 7.35 (s, 1H), 6.27 (s, 1H), 3.18 (m, 1H), 1.32 (s, 6H), 1.19 (d, *J* = 6.4 Hz, 6H).

### Step 4

### 2-(3,5-dichloro-4-((5-isopropyl-6-oxy-1,6-dihydropyridazin-3-yl)oxy)phenyl)-6-methyl-1,2,4-tria zine-3, 5 (2H,4H)-dione

6-(2,6-dichloro-4-(3,3-dimethyl-5-oxy-1,2,4-triazolidin-1-yl)phenoxy)-4-isopropylpyrid azin-3(2H)-one **4d** (500 mg, 1.22 mmol) was dissolved in 10 mL of dioxane solution and added with a catalytic amount of sulfuric acid (0.5 mL) and pyruvic acid **4e** (215 mg, 2.44 mmol), and the reaction solution was heated to 120°C for 5 h. After the completion of the reaction, the reaction solution was added with 50 mL of ice water and extracted with ethyl acetate (50 mL × 3). The organic phases were combined, washed with saturated sodium chloride solution (100 mL), dried over anhydrous sodium sulfate, filtered, and concentrated under reduced pressure. The residue was purified by preparative chromatography to obtain compound 2-(3,5-dichloro-4-((5-isopropyl-6-oxy-1,6-dihydropyridazin-3-yl))oxy)phenyl)-6-methyl-1,2,4-tri azine-3,5(2H,4H)-dione **4** (40 mg, yellow solid), yield: 8%. MS m/z (ESI): 424.0 [M+1]⁺. ¹H NMR (400 MHz, DMSO-*d₆*) δ12.41 (br s, 1H), 12.21 (s, 1H), 7.79 (s, 2H), 7.42 (s, 1H), 3.04 (m, 1H), 2.15 (s, 3H), 1.19 (d, *J* = 6.4 Hz, 6H).

### Example 5

### 2-(3,5-dichloro-4-((5-cyclopentyl-6-oxo-1,6-dihydropyridazin-3-yl)oxy)phenyl)-6-(hydroxymeth yl)-1,2,4-triazine-3,5(2H,4H)-dione

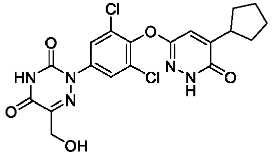

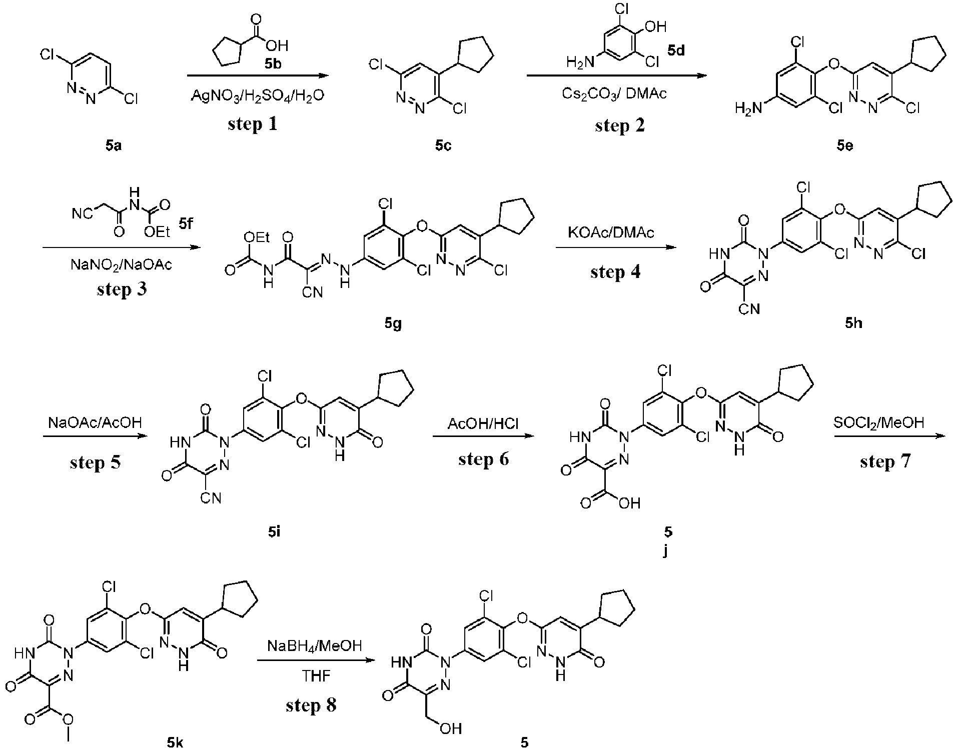

### Step 1

### 3,6-dichloro-4-cyclopentylpyridazine

3,6-dichloropyridazine **5a** (2.5 g, 16.8 mmol) was dissolved in a mixture of 84 mL of water and 3.7 mL of concentrated sulfuric acid and added with silver nitrate (0.575 g, 3.36 mmol) followed by cyclopentanoic acid **5b** (3.25 g, 28.56 mmol), and 40 mL of aqueous solution of ammonium persulfate (13 g, 56.97 mmol) was slowly added dropwise over half an hour at room temperature. After the dropwise addition, the reaction solution was heated to 70°C for 0.5 h of reaction, then cooled to room temperature, adjusted to pH=7 with ammonia water, and extracted with ethyl acetate (25 mL × 3). The organic phases were combined, dried over anhydrous sodium sulfate and filtered, and the filtrate was concentrated under reduced pressure. The resultant residue was purified by silica gel column chromatography to obtain 3,6-dichloro-4-cyclopentylpyridazine **5c** (3.3 g, colorless oily liquid), yield: 91%. MS m/z (ESI): 217.0 [M+1]⁺.

### Step 2

### 3,5-dichloro-4-((6-chloro-5-cyclopentylpyridazin-3-yl)oxy)aniline

3,6-dichloro-4-cyclopentylpyridazine **5c** (1 g, 4.6 mmol) and 4-amino-2,6-dichlorophenol **5d** (0.819 g, 4.69 mmol) were dissolved in 10 mL of N,N dimethylacetamide and added with cesium carbonate (1.73 g, 5.29 mmol). The reaction solution was heated to 110°C and stirred for 3 h, then cooled to room temperature, added with 30 mL of water and extracted with ethyl acetate (30 mL × 3). The organic phases were combined, dried over anhydrous sodium sulfate and filtered, and the filtrate was concentrated under reduced pressure. The resultant residue was purified by silica gel column chromatography to obtain 3,5-dichloro-4-((6-chloro-5-cyclopentylpyridazin-3-yl)oxy)aniline **5e** (1.3 g, white solid), yield: 78%. MS m/z (ESI): 358.0 [M+1]⁺.

### Step 3

### (E)-ethyl(2-cyano-2-(2-(3,5-dichloro-4-((6-chloro-5-cyclopentylpyridazin-3-yl)oxy)phenyl)hydra zine)acetyl)carbamate

3,5-dichloro-4-((6-chloro-5-cyclopentylpyridazin-3-yl)oxy)aniline **5e** (1.30 g, 3.64 mmol) was dissolved in 8 mL of acetic acid and 2 mL of water, cooled down to 0°C , then added with 1 mL of concentrated hydrochloric acid, and slowly added with 4 mL of aqueous solution of sodium nitrite (256 mg, 3.65 mmol) dropwise. After the dropwise addition, the reaction solution was stirred for 10 min, then added with 5 mL of aqueous solution of sodium acetate (830 mg, 10.8 mmol) followed by ethyl (2-cyanoacetic acid) carbamate **5f** (579 mg, 3.65 mmol). After removal of ice bath, the reaction solution was warmed to room temperature for 1 h of reaction. 20 mL of water was added, and the solid was filtered and dried to obtain solid (E)-ethyl(2-cyano-2-(2-(3,5-dichloro-4-((6-chloro-5-cyclopentylpyridazin-3-yl)oxy)phenyl)hydra zine)acetyl)carbamate **5g** (1.20 g, white solid), yield: 63%. MS m/z (ESI): 525.1 [M+1]⁺.

### Step 4

### 2-(3,5-dichloro-4-((6-chloro-5-cyclopentylpyridazin-3-yl)oxy)phenyl)-3,5-dioxo-2,3,4,5-tetrahyd ro-1,2,4-triazine-6-carbonitrile

The compound (E)-ethyl(2-cyano-2-(2-(3,5-dichloro-4-((6-chloro-5-cyclopentylpyridazin-3-yl)oxy)phenyl)hydra zine)acetyl)carbamate **5g** (1.20 g, 2.3 mmol) was dissolved in 10 mL of N,N dimethylacetamide and added with potassium acetate (270 mg, 2.76 mmol), and the reaction solution was heated to 120C and stirred for 3 h, and cooled to room temperature. The reaction solution was added with 20 mL of water and extracted with ethyl acetate (10 mL × 3). The organic phases were combined, dried over anhydrous sodium sulfate and filtered, and the filtrate was concentrated under reduced pressure. The resultant residue was purified by silica gel column chromatography to obtain 2-(3,5-dichloro-4-((6-chloro-5-cyclopentylpyridazin-3-yl)oxy)phenyl)-3,5-dioxo-2,3,4,5-tetrahyd ro-1,2,4-triazine-6-carbonitrile **5h** (1.0 g, white solid), yield: 91%. MS m/z (ESI): 479.1 [M+1]⁺.

### Step 5

### 2-(3,5-dichloro-4-((5-cyclopentyl-6-oxo-1,6-dihydropyridazin-3-yl)oxy)phenyl)-3,5-dioxo-2,3,4, 5-tetrahydro-1,2,4-triazine-6-carbonitrile

The compound 2-(3,5-dichloro-4-((6-chloro-5-cyclopentylpyridazin-3-yl)oxy)phenyl)-3,5-dioxo-2,3,4,5-tetrahyd ro-1,2,4-triazine-6-carbonitrile **5h** (1.00 g, 2.1 mmol) was dissolved in 10 mL of acetic acid and then added with sodium acetate (344 mg, 4.2 mmol), and the reaction solution was heated to 110°C and stirred for 3 h, and cooled to room temperature. The reaction solution was added with 20 mL of water and extracted with ethyl acetate (10 mL × 3). The organic phases were combined, dried over anhydrous sodium sulfate and filtered, and the filtrate was concentrated under reduced pressure. The resultant residue was purified by silica gel column chromatography to obtain 2-(3,5-dichloro-4-((5-cyclopentyl-6-oxo-1,6-dihydropyridazin-3-yl)oxy)phenyl)-3,5-dioxo-2,3,4, 5-tetrahydro-1,2,4-triazine-6-carbonitrile **5i** (823 mg, white solid), yield: 85%. MS m/z(ESI): 461.0 [M+1]⁺. ¹H NMR (400 MHz, DMSO-*d₆*) δ 13.28 (s, 1H), 12.21 (s, 1H), 7.79 (s, 2H), 7.46 (s,1H), 3.02-3.17 (m,1H), 1.97-1.99 (m, 2H), 1.74-1.76 (m, 2H), 1.59-1.66 (m, 4H).

### Step 6

### 2-(3,5-dichloro-4-((5-cyclopentyl-6-oxo-1,6-dihydropyridazin-3-yl)oxy)phenyl)-3,5-dioxo-2,3,4, 5-tetrahydro-1,2,4-triazine-6-carboxylic acid

2-(3,5-dichloro-4-((5-cyclopentyl-6-oxo-1,6-dihydropyridazin-3-yl)oxy)phenyl)-3,5-dio xo-2,3,4,5-tetrahydro-1,2,4-triazine-6-carbonitrile **5i** (823 mg, 1.8 mmol) was dissolved in 10 mL of glacial acetic acid, added with 4 mL of concentrated hydrochloric acid, and heated to 120°C for 5 h of reaction. After the completion of the reaction, the reaction solution was cooled to room temperature, added with 20 mL of water to dilute, stirred and filtered, and the solid was dried to obtain

2-(3,5-dichloro-4-((5-cyclopentyl-6-oxo-1,6-dihydropyridazin-3-yl)oxy)phenyl)-3,5-dioxo-2,3,4, 5-tetrahydro-1,2,4-triazine-6-carboxylic acid **5j** ( 820 mg, pale yellow solid), yield: 95%. MS m/z (ESI): 480.0 [M+1]⁺.

### Step 7

### Methyl 2-(3,5-dichloro-4-((5-cyclopentyl-6-oxo-1,6-dihydropyridazin-3-yl)oxy)phenyl)-3,5-dioxo-2,3,4, 5-tetrahydro-1,2,4-triazine-6-carboxylate

2-(3,5-dichloro-4-((5-cyclopentyl-6-oxo-1,6-dihydropyridazin-3-yl)oxy)phenyl)-3,5-dio xo-2,3,4,5-tetrahydro-1,2,4-triazine-6-carboxylic acid **5j** (820 mg, 1.71 mmol) was dissolved in 10 mL of methanol and added slowly with 2 mL of thionyl chloride under ice bath, and the reaction solution was heated to 80°C for 5 h. After the completion of the reaction, the reaction solution was concentrated under reduced pressure, and the residual solid was slurried with petroleum ether: ethyl acetate (20:1) and filtered. The solid was dried to obtain methyl 2-(3,5-dichloro-4-((5-cyclopentyl-6-oxo-1,6-dihydropyridazin-3-yl)oxy)phenyl)-3,5-dioxo-2,3,4, 5-tetrahydro-1,2,4-triazine-6-carboxylate **5k** (819 mg, pale yellow solid), yield: 97%. MS m/z (ESI): 494.0 [M+1]⁺.

### Step 8

### 2-(3,5-dichloro-4-((5-cyclopentyl-6-oxo-1,6-dihydropyridazin-3-yl)oxy)phenyl)-6-(hydroxymeth yl)-1,2,4-triazine-3,5(2H,4H)-dione

Methyl 2-(3,5-dichloro-4-((5-cyclopentyl-6-oxo-1,6-dihydropyridazin-3-yl)oxy)phenyl)-3,5-dioxo-2,3,4, 5-tetrahydro-1,2,4-triazine-6-carboxylate **5k** (819 mg, 1.66 mmol) was dissolved in 10 mL of tetrahydrofuran, then added with sodium borohydride ( 315 mg, 8.3 mmol), and heated to 60°C. Methanol was slowly added dropwise until no gas was generated, and the reaction solution was heated for 3 h of reaction. After the completion of the reaction, the reaction solution was added with 20 mL of water, adjusted to pH=6 with 3M dilute hydrochloric acid, and extracted with ethyl acetate (30 mL × 3). The organic phases were combined, washed with saturated sodium chloride solution (200 mL), dried over anhydrous sodium sulfate, filtered and concentrated under reduced pressure. The residue was purified by silica gel column chromatography to obtain 2-(3,5-dichloro-4-((5-cyclopentyl-6-oxo-1,6-dihydropyridazin-3-yl)oxy)phenyl)-6-(hydroxymeth yl)-1,2,4-triazine-3,5(2H,4H)-dione **5** (688 mg, white solid), yield: 89%. MS m/z (ESI): 466.0 [M+1]⁺.

### Example 6

### 2-(3,5-dichloro-4-((5-isopropyl-6-oxo-1,6-dihydropyridazin-3-yl)oxy)phenyl)-6-(trifluoromethyl) -1,2,4-triazine-3,5(2H,4H)-dione

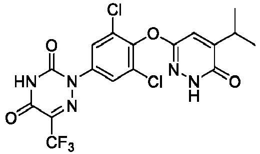

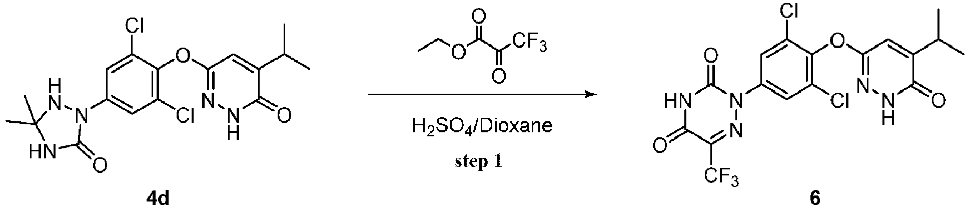

### Step 1

### 2-(3,5-dichloro-4-((5-isopropyl-6-oxo-1,6-dihydropyridazin-3-yl)oxy)phenyl)-6-(trifluoromethyl) -1,2,4-triazine-3,5(2H,4H)-dione

6-(2,6-dichloro-4-(3,3-dimethyl-5-oxo-1,2,4-triazolidin-1-yl)phenoxy)-4-isopropylpyrid azin-3(2H)-one **4a** (200 mg, 0.486 mmol) was dissolved in 4 mL of dioxane, added with ethyl trifluoropyruvate (82 mg, 0.486 mmol) followed by 2 drops of concentrated sulfuric acid, and heated to 100°C for 5 h of reaction. The reaction solution was cooled to room temperature, concentrated under reduced pressure to remove the solvent, and purified by preparative chromatography to obtain 2-(3,5-dichloro-4-((5-isopropyl-6-oxo-1,6-dihydropyridazin-3-yl)oxy)phenyl)-6-(trifluoromethyl) -1,2,4-triazine-3,5(2H,4H)-dione **6** (1.5 mg, white solid), yield: 1%. MS m/z (ESI): 478.0 [M+1]⁺.
¹H NMR (400 MHz, DMSO-*d₆*) δ 12.21(s, 1H), 7.78 (s, 2H), 7.45 (s, 1H), 3.06-3.03(m, 1H), 1.20 (d, *J* = 4.0 Hz, 6H).

### Example 7

### 2-(3,5-dichloro-4-((5-isopropyl-6-oxo-1,6-dihydropyridazin-3-yl)oxy)phenyl)-6-methoxy-1,2,4-tr iazine-3, 5 (2H,4H)-dione

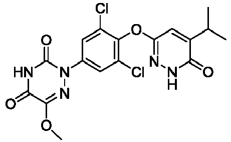

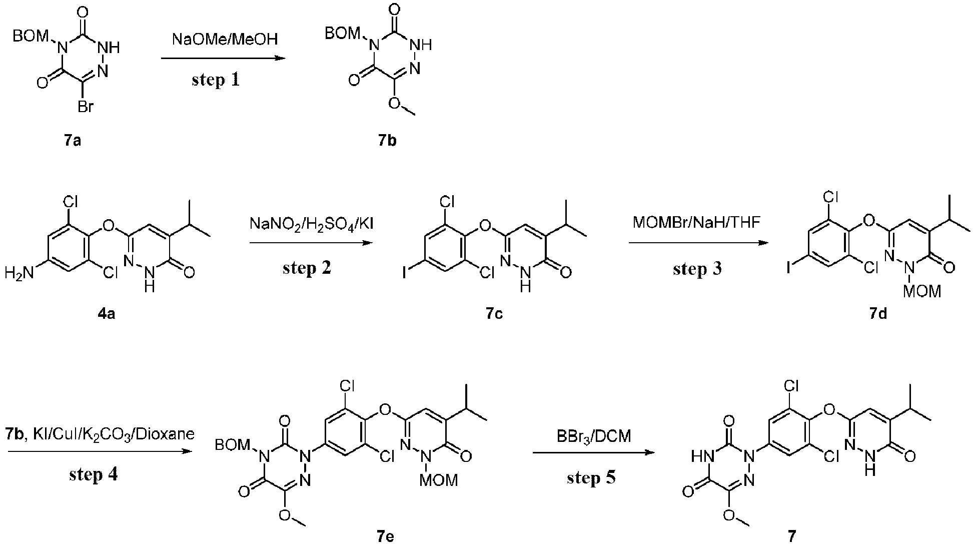

### Step 1

### 4-((benzyloxy)methyl)-6-methoxy-1,2,4-triazine-3,5(2H,4H)-dione

4-((benzyloxy)methyl)-6-bromo-1,2,4-triazine-3,5(2H,4H)-dione **7a** (1 g, 3.21 mmol) (see WO 2010006962 for the synthesis method) was added to 5 mL of methanol, then added with sodium methoxide (434 mg, 8.04 mmol), and microwaved at 100°C for 4 h of reaction. The reaction solution was concentrated under reduced pressure to remove the solvent, added with 10 mL of IN dilute hydrochloric acid, and extracted with ethyl acetate (10 mL × 3). The organic phases were combined, washed with saturated brine once, dried over anhydrous sodium sulfate, filtered and concentrated. The residue was purified by column chromatography by rinsing with petroleum ether: ethyl acetate from 1:0 to 2:1 to obtain 4-((benzyloxy)methyl)-6-methoxy-1,2,4-triazine-3,5(2H,4H)-dione 7b (230 mg, white solid), yield: 27 %. MS m/z (ESI): 262.0 [M-1]⁻.

### Step 2

### 6-(2,6-dichloro-4-iodophenoxy)-4-isopropylpyridazin-3(2H)-one

6-(4-amino-2,6-dichlorophenoxy)-4-isopropylpyridazin-3(2H)-one 4a (950 mg, 3.02 mmol) (see WO 2014043706 for the synthesis method) was dissolved in 20 mL of dilute sulfuric acid (20%). After the system was cooled to 0°C, 5 mL of aqueous solution of sodium nitrite (0.25 g, 3.33 mmol) was slowly added dropwise. The system was maintained at 0°C - 5°C, and was added dropwise with 15 mL of aqueous potassium iodide solution (0.55 g, 3.33 mmol) after 20 min of reaction. The system was then placed at room temperature for reaction, and was detected by TLC for the completion of the reaction after 3 h. The system was extracted with ethyl acetate three times. After concentrated, the organic phase was slurried to obtain a suspension, which was filtered with suction to obtain a filter cake. After drying, 6-(2,6-dichloro-4-iodophenoxy)-4-isopropylpyridazin-3(2H)-one **7c** (750 mg, pale yellow solid) was obtained, yield: 58 %. MS m/z (ESI): 425.0 [M+1]⁺.

### Step 3

### 6-(2,6-dichloro-4-iodophenoxy)-4-isopropyl-2-(methoxymethyl)pyridazin-3(2H)-one

6-(2,6-dichloro-4-iodophenoxy)-4-isopropylpyridazin-3(2H)-one **7c** (563 mg, 1.33 mmol) was added to 12 mL of anhydrous tetrahydrofuran. Under nitrogen protection, sodium hydride (64 mg, 60%, 1.59 mmol) was added at 0°C . After the reaction was warmed to room temperature for 10 min, methoxymethyl bromide (332 mg, 2.66 mmol) was added, and after 10 min of reaction, 10 mL of saturated brinewas added. The reaction solution was extracted with ethyl acetate (10 mL × 2), dried over anhydrous sodium sulfate, filtered and concentrated. The residue was purified by column chromatography by rinsing with petroleum ether: ethyl acetate from 1:0 to 5:1 to obtain 6-(2,6-dichloro-4-iodophenoxy)-4-isopropyl-2-(methoxymethyl)pyridazin-3(2H)-one **7d** (350 mg, white solid), yield: 56%. MS m/z (ESI): 469.0 [M+1]⁺.

### Step 4

### 4-((benzyloxy)methyl)-2-(3,5-dichloro-4-((5-isopropyl-1-(methoxymethyl)-6-oxy-1,6-dihydropyr idazin-3-yl)oxy)phenyl)-6-methoxy-1,2,4-triazine-3,5(2H,4H)-dione

4-((benzyloxy)methyl)-6-methoxy-1,2,4-triazine-3,5(2H,4H)-dione **7b** (90 mg, 0.34 mmol) was dissolved in 2 mL of dioxane, then added with 6-(2,6-dichloro-4-iodophenoxy)-4-isopropyl-2-(methoxymethyl)pyridazine-3(2H)-one **7d** (160 mg, 0.34 mmol) followed by trans-(1R,2R)-N,N'-dimethyl 1,2-cyclohexanediamine (10 mg, 0.068 mmol) and potassium iodide (57 mg, 0.34 mmol), cuprous iodide (65 mg, 0.34 mmol), and potassium carbonate (95 mg, 0.68 mmol), and then microwaved at 130°C for 2 h of reaction. The reaction solution was added with 10 mL of ethyl acetate and 10 mL of water, and the layers were separated. The aqueous phase was extracted with 10 mL of ethyl acetate once, and the organic phases were combined, washed with 10 mL of saturated brine once, dried over anhydrous sodium sulfate, filtered and concentrated. The residue was purified by column chromatography by rinsing with petroleum ether: ethyl acetate from 1:0 to 5:1 to obtain 4-((benzyloxy)methyl)-2-(3,5-dichloro-4-((5-isopropyl-1-(methoxymethyl)-6-oxy-1,6-dihydropyr idazin-3-yl)oxy)phenyl)-6-methoxy-1,2,4-triazine-3,5(2H,4H)-dione **7e** (160 mg, colorless oil), yield: 77%. MS m/z (ESI): 604.0 [M+1]⁺.

### Step 5

### 2-(3,5-dichloro-4-((5-isopropyl-6-oxo-1,6-dihydropyridazin-3-yl)oxy)phenyl)-6-methoxy-1,2,4-tr iazine-3, 5 (2H,4H)-dione

4-((benzyloxy)methyl)-2-(3,5-dichloro-4-((5-isopropyl-1-(methoxymethyl)-6-oxy-1,6-d ihydropyridazin-3-yl)oxy)phenyl)-6-methoxy-1,2,4-triazine-3,5(2H,4H)-dione **7e** (80 mg, 0.13 mmol) was dissolved in 2 mL of dichloromethane. Under nitrogen protection, boron tribromide (0.40 mL, 1 mol/L) was added to react overnight at room temperature. 1 mL of methanol was added to quench the reaction, and then the solvent was removed by concentration under reduced pressure. Then 1 mL of methanol and 1 mL of concentrated sulfuric acid were added, and the reaction was carried out at 100°C 1 h. After the completion of the reaction, the reaction solution was cooled to room temperature, added dropwise to ice water, adjusted to pH=5~6 with saturated aqueous potassium carbonate solution, and extracted with ethyl acetate twice (20 mL × 2). The organic phases were combined, washed with 10 mL of saturated brine once, dried over anhydrous sodium sulfate, filtered and concentrated. The residue was purified by preparative chromatography to obtain 2-(3,5-dichloro-4-((5-isopropyl-6-oxo-1,6-dihydropyridazin-3-yl)oxy)phenyl)-6-methoxy-1,2,4-tr iazine-3,5(2H,4H)-dione **7** (10 mg, pale yellow solid), yield: 17%. MS m/z (ESI): 440.0 [M+1]⁺. ¹H NMR (400 MHz, DMSO-*d₆*) δ 12.50 (s, 1H), 12.20 (s, 1H), 7.85 (s, 2H), 7.43 (s, 1H), 3.84 (s, 3H), 3.01-3.07 (m, 1H), 1.19-1.20 (d, *J* = 4.0 Hz, 6H).

### Example 8

### 2-(3,5-dichloro-4-((5-cyclohexyl-6-oxo-1,6-dihydropyridazin-3-yl)oxy)phenyl)-6-(hydroxymethy 1)-1,2,4-triazine-3,5(2H,4H)-dione

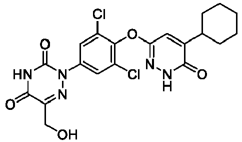

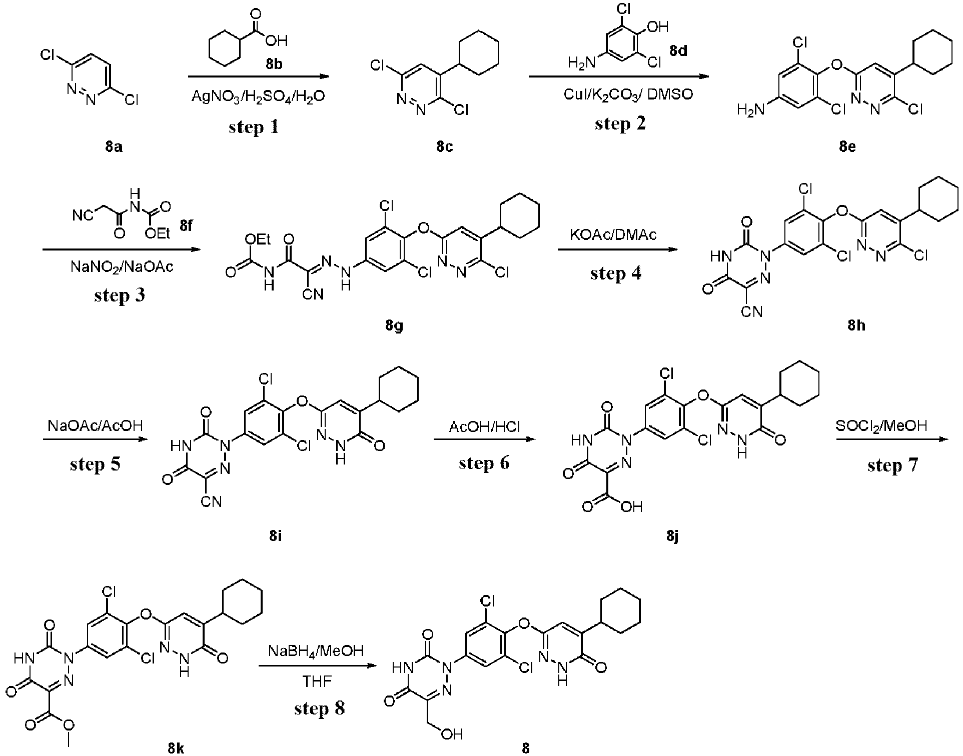

### Step 1

### 3,6-dichloro-4-cyclohexylpyridazine

Under nitrogen protection, 3,6-dichloropyridazine **8a** (1.49 g, 10 mmol), cyclohexanecarboxylic acid (4.49 g, 35 mmol), silver nitrate (0.17 g, 1 mmol) and concentrated sulfuric acid (1.81 mL, 30 mmol) were added to 300 mL of water and heated to 70°C. Ammonium persulfate (2.28 g, 10 mmol) was dissolved in 100 mL of water, and this solution was added dropwise to the above reaction solution within 10 min. The reaction was carried out at 70C for 24 h. The reaction system was cooled to room temperature, adjusted to pH = 8 with ammonia water and extracted with dichloromethane (100 mL × 3). The organic phases were combined, washed with IN sodium hydroxide solution once, dried over anhydrous sodium sulfate, filtered, concentrated, and purified by silica gel column by rinsing with petroleum ether: ethyl acetate from 1:0 to 5:1 to obtain 3,6-dichloro-4-cyclohexylpyridazine **8b** (829 mg, colorless oil), yield: 36%. MS m/z (ESI): 231 [M+1]⁺.

### Step 2

### 3,5-dichloro-4-((6-chloro-5-cyclohexylpyridazin-3-yl)oxy)aniline

3,6-dichloro-4-cyclohexylpyridazine **3b** (829 mg, 3.6 mmol), 4-amino-2,6-dichlorophenol **8d** (642 mg, 3.6 mmol), potassium carbonate (2.0 g, 14.4 mmol) and cuprous iodide (686 mg, 3.6 mmol) were added to 13 mL of dimethyl sulfoxide and replaced with nitrogen three times. Then the reaction solution was reacted at 120°C for 16 h, added with 100 mL of water, and extracted with ethyl acetate (20 mL × 3). The organic phases were combined, washed with saturated brine once, dried over anhydrous sodium sulfate, filtered, concentrated, and then purified by silica gel column by rinsing with petroleum ether: ethyl acetate from 1:0 to 3:1 to obtain 3,5-dichloro-4-((6-chloro-5-cyclohexylpyridazin-3-yl)oxy)aniline **8e** (678 mg, white solid), yield: 50%. MS m/z (ESI): 373 [M+1]⁺.

### Step 3

### (E)-ethyl(2-cyano-2-(2-(3,5-dichloro-4-((6-chloro-5-cyclohexylpyridazin-3-yl)oxy)phenyl)hydraz ine)acetyl) carbamate

3,5-dichloro-4-((6-chloro-5-cyclohexylpyridazin-3-yl)oxy)aniline **8e** (2 g, 5.376 mmol) was dissolved in a mixed solvent (64 mL acetic acid, 16 mL water and 8 mL of concentrated hydrochloric acid), cooled to 0°C, added with sodium nitrite (445 mg, 6.45 mmol) at this temperature, and reacted for 10 min. Sodium acetate (1.33 g, 16.1 mmol) was then added, and reacted at 0°C for another 10 min. Then ethyl (2-cyanoacetic acid) carbamate **8f** (1.0 g, 6.45 mmol) was added and reacted at room temperature for 2 h. The reaction solution was added with 50 mL of water and filtered, and the filter cake was rinsed with water once and dried in a vacuum oven to obtain (E)-ethyl(2-cyano-2-(2-(3,5-dichloro-4-((6-chloro-5-cyclohexylpyridazin-3-yl)oxy)phenyl)hydraz ine)acetyl) carbamate **8g** (1.8 g, pale yellow solid), yield: 62%. MS m/z (ESI): 540 [M+1]⁺.

### Step 4

### 2-(3,5-dichloro-4-((6-chloro-5-cyclohexylpyridazin-3-yl)oxy)phenyl)-3,5-dioxo-2,3,4,5-tetrahydr o-1,2,4-triazine-6-carbonitrile

(E)-ethyl(2-cyano-2-(2-(3,5-dichloro-4-((6-chloro-5-cyclohexylpyridazin-3-yl)oxy)phen yl)hydrazine)acetyl) carbamate **8g** (1.8 g, 3.34 mmol) was dissolved in 36 mL of N,N-dimethylacetamide and added with potassium acetate (492 mg, 5 mmol). This reaction solution was reacted at 120°C for 2 h, then added to 100 mL of water and filtered, and the resultant solid was dried in a vacuum drying oven to obtain 2-(3,5-dichloro-4-((6-chloro-5-cyclohexylpyridazin-3-yl)oxy)phenyl)-3,5-dioxo-2,3,4,5-tetrahydr o-1,2,4-triazine-6-carbonitrile **8h** (1.65 g, pale yellow solid), yield: 100%. MS m/z (ESI): 494 [M+1]⁺.

### Step 5

### 2-(3,5-dichloro-4-((5-cyclohexyl-6-oxo-1,6-dihydropyridazin-3-yl)oxy)phenyl)-3,5-dioxo-2,3,4,5 -tetrahydro-1,2,4-triazine-6-carbonitrile

2-(3,5-dichloro-4-((6-chloro-5-cyclohexylpyridazin-3-yl)oxy)phenyl)-3,5-dioxo-2,3,4,5-tetrahydro-1,2,4-triazine-6-carbonitrile **8h** (1.66 g, 3.37 mmol) was added to 30 mL of glacial acetic acid and added with sodium acetate (552 mg, 6.73 mmol). This solution was heated to 120°C and reacted for 24 h. The solution was added with 100 mL of water and filtered, and the filter cake was dried in a vacuum drying oven and then slurried with 50 mL of ethyl acetate to obtain 2-(3,5-dichloro-4-((5-cyclohexyl-6-oxo-1,6-dihydropyridazin-3-yl)oxy)phenyl)-3,5-dioxo-2,3,4,5 -tetrahydro-1,2,4-triazine-6-carbonitrile **8i** (1.09 g, pale yellow solid), yield: 68%.

¹H NMR (400 MHz, DMSO-*d₆*) δ 13.27 (s, 1H), 12.23 (s, 1H), 7.78 (s, 2H), 7.39 (s,1H), 2.67-2.80 (m,1H), 1.65-1.90 (m, 5H),1.15-1.45(m, 5H). MS m/z (ESI): 475[M+1]⁺.

### Step 6

### 2-(3,5-dichloro-4-((5-cyclohexyl-6-oxo-1,6-dihydropyridazin-3-yl)oxy)phenyl)-3,5-dioxo-2,3,4,5 -tetrahydro-1,2,4-triazine-6-carboxylic acid

2-(3,5-dichloro-4-((5-cyclohexyl-6-oxo-1,6-dihydropyridazin-3-yl)oxy)phenyl)-3,5-dio xo-2,3,4,5-tetrahydro-1,2,4-triazine-6-carbonitrile **8i** (500 mg, 1.05 mmol) was added to a mixed solvent (4 mL of concentrated hydrochloric acid and 10 mL of glacial acetic acid) and reacted at 120°C for 5 h in a sealed tube. After cooled to room temperature, the reaction solution was added with 20 mL of water and filtered, and the filter cake was dried in a vacuum drying oven to obtain 2-(3,5-dichloro-4-((5-cyclohexyl-6-oxo-1,6-dihydropyridazin-3-yl)oxy)phenyl)-3,5-dioxo-2,3,4,5 -tetrahydro-1,2,4-triazine-6-carboxylic acid **8j** (380 mg, pale yellow solid), yield: 73%. MS m/z (ESI): 494 [M+1]⁺.

### Step 7

### Methyl 2-(3,5-dichloro-4-((5-cyclohexyl-6-oxo-1,6-dihydropyridazin-3-yl)oxy)phenyl)-3,5-dioxo-2,3,4,5 -tetrahydro-1,2,4-triazine-6-carboxylate

2-(3,5-dichloro-4-((5-cyclohexyl-6-oxo-1,6-dihydropyridazin-3-yl)oxy)phenyl)-3,5-dio xo-2,3,4,5-tetrahydro-1,2,4-triazine-6-carboxylic acid **8j** (380 mg, 0.77 mmol) was suspended in 10 mL of methanol, added dropwise with 1 mL of thionyl chloride under ice-water bath, refluxed for 3 h of reaction, and concentrated under reduced pressure to obtain methyl 2-(3,5-dichloro-4-((5-cyclohexyl-6-oxo-1,6-dihydropyridazin-3-yl)oxy)phenyl)-3,5-dioxo-2,3,4,5 -tetrahydro-1,2,4-triazine-6-carboxylate **8k** (380 mg, pale yellow solid), yield: 97%. MS m/z (ESI): 508 [M+1]⁺.

### Step 8

### 2-(3,5-dichloro-4-((5-cyclohexyl-6-oxo-1,6-dihydropyridazin-3-yl)oxy)phenyl)-6-(hydroxymethy l)-1,2,4-triazine-3,5(2H,4H)-dione

Methyl 2-(3,5-dichloro-4-((5-cyclohexyl-6-oxo-1,6-dihydropyridazin-3-yl)oxy)phenyl)-3,5-dioxo-2,3,4,5 -tetrahydro-1,2,4-triazine-6-carboxylate **8k** (380 mg, 0.749 mmol) was dissolved in 20 mL of tetrahydrofuran and added with sodium borohydride (57 mg, 1.5 mmol). 5 mL of methanol was then slowly added dropwise under reflux, and the reaction was continued under reflux for 5 h. The reaction solution was added with saturated ammonium chloride solution to quench the reaction (50 ml) and filtered, and the filter cake was dried in a vacuum drying oven to obtain 2-(3,5-dichloro-4-((5-cyclohexyl-6-oxo-1,6-dihydropyridazin-3-yl)oxy)phenyl)-6-(hydroxymethy l)-1,2,4-triazine-3,5(2H,4H)-dione **8** (258 mg, pale yellow solid), yield: 72%. MS m/z (ESI): 480 [M+1]⁺.

### Example 9

### 2-(3,5-dichloro-4-((5-cyclohexyl-6-oxo-1,6-dihydropyridazin-3-yl)oxy)phenyl)-6-(fluoromethyl)-1,2,4-triazine-3,5(2H,4H)-dione

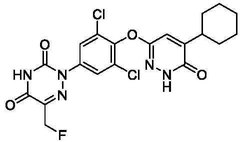

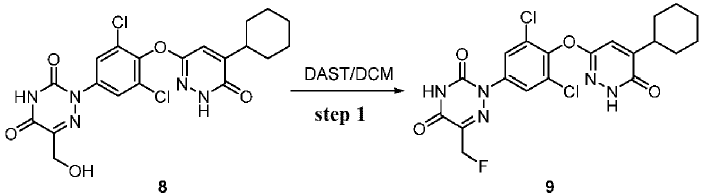

In step 1, 2-(3,5-dichloro-4-((5-cyclohexyl-6-oxo-1,6-dihydropyridazin-3-yl)oxy)phenyl)-6-(hydroxymethy l)-1,2,4-triazine-3,5(2H,4H)-dione **8** (70 mg, 0.15 mmol) was suspended in 5 mL of dichloromethane, slowly added dropwise with diethylaminosulfur trifluoride (228 mg, 1.42 mmol) under ice-water bath, and warmed to room temperature for 15 min of reaction. The reaction solution was added with ice water to quench the reaction, extracted with dichloromethane (10 mL×3), dried over anhydrous sodium sulfate, filtered, concentrated, and purified by preparative chromatography to obtain 2-(3,5-dichloro-4-((5-cyclohexyl-6-oxo-l,6-dihydropyridazin-3-yl)oxy)phenyl)-6-(fluoromethyl)-1,2,4-triazine-3,5(2H,4H)-dione **9** (10 mg, white solid), yield: 14%. MS m/z (ESI): 482 [M+1]⁺. ¹H NMR (400 MHz, DMSO-*d₆*) δ 12.67 (s,lH), 12.20 (s, 1H), 7.81 (s, 2H), 7.40 (s, 1H), 5.19-5.39 (d, *J* = 44.0 Hz, 2H), 1.93-2.05 (m, 1H), 1.67-1.88 (m, 5H), 1.31-1.40 (m, 5H).

### Example 10

### 2-(3,5-dichloro-4-((5-cyclopentyl-6-oxo-1,6-dihydropyridazin-3-yl)oxy)phenyl)-6-(fluoromethyl) -1,2,4-triazine-3,5(2H,4H)-dione

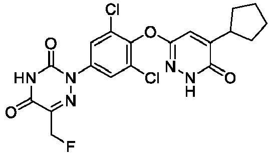

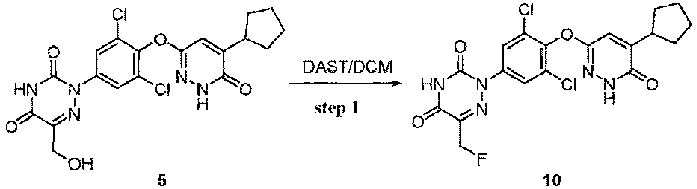

### Step 1

2-(3,5-dichloro-4-((5-cyclopentyl-6-oxo-1,6-dihydropyridazin-3-yl)oxy)phenyl)-6-(hydr oxymethyl)-1,2,4-triazine-3,5(2H,4H)-dione **5** (80 mg, 0.17 mmol) was dissolved in 5 mL of dichloromethane and added with DAST (34 mg, 1.05 mmol) under ice bath, and the reaction solution was reacted at this temperature for 30 min. After the completion of the reaction, the reaction solution was added to 10 mL of ice water and extracted with dichloromethane (5 mL × 3). The organic phases were combined, washed with saturated sodium chloride solution, dried over anhydrous sodium sulfate, filtered and concentrated under reduced pressure. The residue was purified by preparative chromatography to obtain 2-(3,5-dichloro-4-((5-cyclopentyl-6-oxo-1,6-dihydropyridazin-3-yl)oxy)phenyl)-6-(fluoromethyl) -1,2,4-triazine-3,5(2H,4H)-dione **10** (10 mg, white solid), yield: 12%. MS m/z (ESI): 468.0 [M+1] ⁺. ¹H NMR (400 MHz, DMSO-*d₆*) δ 13.29 (s, 1H), 12.21 (s, 1H), 7.82 (s, 2H), 7.48 (s, 1H), 5.31 (d, *J* = 44.0 Hz, 1H), 5.25(s, 1H), 3.14-3.20 (m,1H), 1.98-2.00 (m, 2H), 1.76-1.78 (m, 2H), 1.62-1.68 (m, 4H).

### Example 11

### 2-(3,5-dichloro-4-((5-cyclopropyl-6-oxo-1,6-dihydropyridazin-3-yl)oxy)phenyl)-6-(hydroxymeth yl)-1,2,4-triazine-3,5(2H,4H)-dione

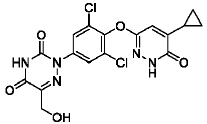

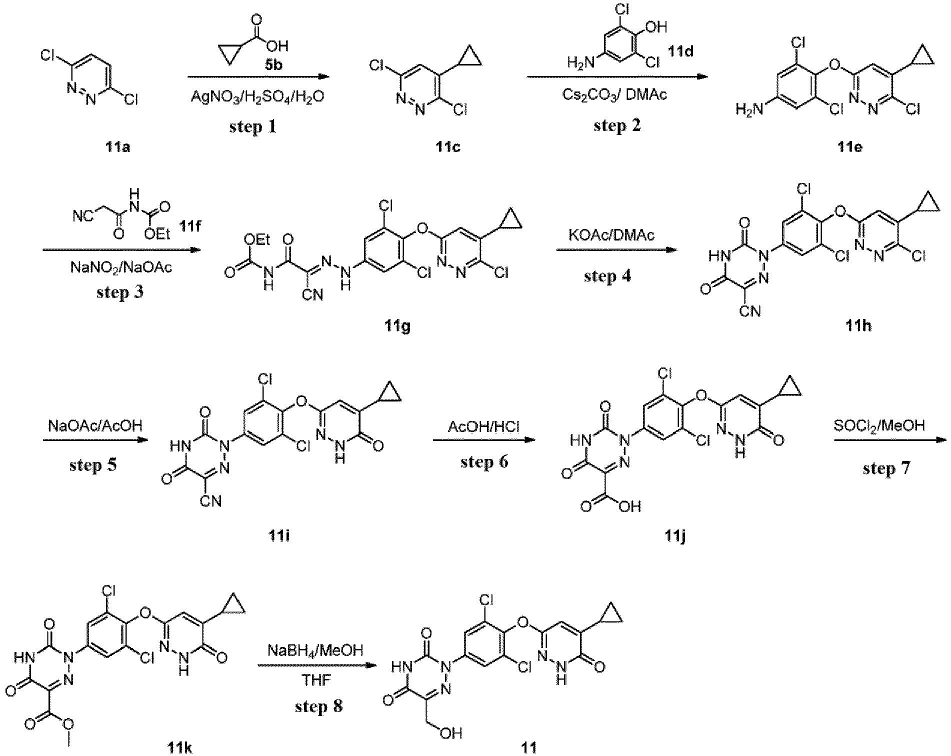

### Step 1

### 3,6-dichloro-4-cyclopropylpyridazine

3,6-dichloropyridazine **11a** (5 g, 33.5 mmol) was dissolved in a mixture of 160 mL of water and 7.4 mL of concentrated sulfuric acid, then added with silver nitrate (1.14 g, 6.7 mmol) followed by cyclopropionic acid **11b** (4.89 g, 57.0 mmol), and slowly added with 60 mL of aqueous solution of ammonium persulfate (25.97 g, 113.9 mmol) dropwise at room temperature within half an hour. After the dropwise addition, the reaction solution was heated to 70°C for 0.5 h of reaction, then cooled to room temperature, adjusted to pH=7 with ammonia water and extracted with ethyl acetate (100 mL × 3). The organic phases were combined, dried over anhydrous sodium sulfate and filtered, and the filtrate was concentrated under reduced pressure. The resultant residue was purified by silica gel column chromatography to obtain 3,6-dichloro-4-cyclopropylpyridazine **11c** (2.02 g, colorless oily liquid), yield: 33%. MS m/z (ESI): 189.0 [M+1]⁺.

### Step 2

### 3,5-dichloro-4-((6-chloro-5-cyclopropylpyridazin-3-yl)oxy)aniline

3,6-dichloro-4-cyclopentylpyridazine **11c** (2.02 g, 10.7 mmol) and 4-amino-2,6-dichlorophenol **11d** (2.09 g, 11.1 mmol) were dissolved in 20 mL N,N dimethylacetamide, added with cesium carbonate (4.17 g, 12.8 mmol), heated to 110°C and stirred for 3 h. The reaction solution was cooled to room temperature, added with 50 mL of water and extracted with ethyl acetate (30 mL × 3). The organic phases were combined, dried over anhydrous sodium sulfate and filtered, and the filtrate was concentrated under reduced pressure. The resultant residue was purified by silica gel column chromatography to obtain 3,5-dichloro-4-((6-chloro-5-cyclopropylpyridazin-3-yl)oxy)aniline **11e** (1.7 g, white solid), yield: 56%. MS m/z (ESI): 329.9 [M+1]⁺.

### Step 3

### (E)-ethyl(2-cyano-2-(2-(3,5-dichloro-4-((6-chloro-5-cyclopropylpyridazin-3-yl)oxy)phenyl)hydra zine)acetyl)carbamate

3,5-dichloro-4-((6-chloro-5-cyclopropylpyridazin-3-yl)oxy)aniline **11e** (1.13 g, 3.64 mmol) was dissolved in 8 mL of acetic acid and 2 mL of water, cooled to 0°C , added with 1 mL of concentrated hydrochloric acid, and then slowly added with 4 mL of aqueous solution of sodium nitrite (241 mg, 3.71 mmol) dropwise. After the dropwise addition, the reaction solution was stirred for 10 min, then added with 5 mL of aqueous solution of sodium acetate (781 mg, 10.1 mmol) followed by ethyl (2-cyanoacetic acid) carbamate **11f** (546 mg, 3.71 mmol). The ice bath was removed, and the reaction solution was warmed to room temperature for 1 h of reaction. 20 mL of water was added, and the solid was filtered and dried to obtain solid (E)-ethyl(2-cyano-2-(2-(3,5-dichloro-4-((6-chloro-5-cyclopropylpyridazin-3-yl)oxy)phenyl)hydra zine)acetyl)carbamate **11g** (1.33 g, white solid), yield: 73%. MS m/z (ESI): 496.9 [M+1]⁺.

### Step 4

### 2-(3,5-dichloro-4-((6-chloro-5-cyclopropylpyridazin-3-yl)oxy)phenyl)-3,5-dioxo-2,3,4,5-tetrahyd ro-1,2,4-triazine-6-carbonitrile

Compound (E)-ethyl(2-cyano-2-(2-(3,5-dichloro-4-((6-chloro-5-cyclopropylpyridazin-3-yl)oxy)phenyl)hydra zine)acetyl)carbamate **11g** (1.33 g, 2.7 mmol) was dissolved in 10 mL of N,N dimethylacetamide, added with potassium acetate (314 mg, 3.21 mmol), heated to 120C and stirred for 3 h. The reaction solution was cooled to room temperature, added with 20 mL of water and extracted with ethyl acetate (10 mL × 3). The organic phases were combined, dried over anhydrous sodium sulfate and filtered, and the filtrate was concentrated under reduced pressure. The resultant residue was purified by silica gel column chromatography to obtain 2-(3,5-dichloro-4-((6-chloro-5-cyclopropylpyridazin-3-yl)oxy)phenyl)-3,5-dioxo-2,3,4,5-tetrahyd ro-1,2,4-triazine-6-carbonitrile **11h** (1.0 g, white solid), yield: 82%. MS m/z (ESI): 450.9 [M+1]⁺.

### Step 5

### 2-(3,5-dichloro-4-((5-cyclopropyl-6-oxo-1,6-dihydropyridazin-3-yl)oxy)phenyl)-3,5-dioxo-2,3,4, 5-tetrahydro-1,2,4-triazine-6-carbonitrile

Compound 2-(3,5-dichloro-4-((6-chloro-5-cyclopropylpyridazin-3-yl)oxy)phenyl)-3,5-dioxo-2,3,4,5-tetrahyd ro-1,2,4-triazine-6-carbonitrile **11h** (600 mg, 1.3 mmol) was dissolved in 10 mL of acetic acid, added with sodium acetate (120 mg, 1.47 mmol), heated to 110°C and stirred for 3 h. The reaction solution was cooled to room temperature, added with 20 mL of water and extracted with ethyl acetate (10 mL × 3). The organic phases were combined, dried over anhydrous sodium sulfate and filtered, and the filtrate was concentrated under reduced pressure. The resultant residue was purified by silica gel column chromatography to obtain 2-(3,5-dichloro-4-((5-cyclopropyl-6-oxo-1,6-dihydropyridazin-3-yl)oxy)phenyl)-3,5-dioxo-2,3,4, 5-tetrahydro-1,2,4-triazine-6-carbonitrile **11i** (440 mg, white solid), yield: 75%. MS m/z (ESI): 433.0 [M+1]⁺. ¹H NMR (400 MHz, DMSO-*d₆*) δ 12.21 (s, 1H), 7.77 (s, 2H), 7.18 (s, 1H), 2.08-2.17 (m, 1H), 1.06-1.15 (m, 2H), 0.95-1.03 (m, 2H).

### Step 6

### 2-(3, 5-dichloro-4-((5-cyclopropyl-6-oxo-1,6-dihydropyridazin-3-yl)oxy)phenyl)-3, 5-dioxo-2,3,4, 5-tetrahydro-1,2,4-triazine-6-carboxylic acid

2-(3,5-dichloro-4-((5-cyclopropyl-6-oxo-1,6-dihydropyridazin-3-yl)oxy)phenyl)-3,5-dio xo-2,3,4,5-tetrahydro-1,2,4-triazine-6-carbonitrile **11i** (440 mg, 1.0 mmol) was dissolved in 10 mL of glacial acetic acid, added with 4 mL of concentrated hydrochloric acid, and heated to 120°C for 5 h of reaction. After the completion of the reaction, the reaction solution was cooled to room temperature, added with 20 mL of water to dilute, stirred and filtered, and the solid was dried to obtain 2-(3,5-dichloro-4-((5-cyclopropyl-6-oxo-1,6-dihydropyridazin-3-yl)oxy)phenyl)-3,5-dioxo-2,3,4, 5-tetrahydro-1,2,4-triazine-6-carboxylic acid **11j** ( 352 mg, pale yellow solid), yield: 80%. MS m/z (ESI): 451.9 [M+1]⁺.

### Step 7

### Methyl 2-(3,5-dichloro-4-((5-cyclopropyl-6-oxo-1,6-dihydropyridazin-3-yl)oxy)phenyl)-3,5-dioxo-2,3,4, 5-tetrahydro-1,2,4-triazine-6-carboxylate

2-(3,5-dichloro-4-((5-cyclopropyl-6-oxo-1,6-dihydropyridazin-3-yl)oxy)phenyl)-3,5-dio xo-2,3,4,5-tetrahydro-1,2,4-triazine-6-carboxylic acid **11j** (268 mg, 0.59 mmol) was dissolved in 10 mL of methanol, added slowly with 2 mL of thionyl chloride under ice bath, and heated to 80°C for 5 h of reaction. After the completion of the reaction, the reaction solution was concentrated under reduced pressure, and the residual solid was slurried with petroleum ether: ethyl acetate (20:1) and filtered. The solid was dried to obtain methyl 2-(3,5-dichloro-4-((5-cyclopropyl-6-oxo-1,6-dihydropyridazin-3-yl)oxy)phenyl)-3,5-dioxo-2,3,4, 5-tetrahydro-1,2,4-triazine-6-carboxylate **11k** (279 mg, pale yellow solid), yield: 100%. MS m/z (ESI): 466.0 [M+1]⁺.

### Step 8

### 2-(3, 5-dichloro-4-((5-cyclopropyl-6-oxo-1,6-dihydropyridazin-3 -yl)oxy)phenyl)-6-(hydroxymeth yl)-1,2,4-triazine-3,5(2H,4H)-dione

Methyl 2-(3,5-dichloro-4-((5-cyclopropyl-6-oxo-1,6-dihydropyridazin-3-yl)oxy)phenyl)-3,5-dioxo-2,3,4, 5-tetrahydro-1,2,4-triazine-6-carboxylate **11k** (279 mg, 0.6 mmol) was dissolved in 50 mL of tetrahydrofuran, added with sodium borohydride (68 mg, 1.8 mmol), and heated to 60°C. Methanol was slowly added dropwise until no gas was generated, and the reaction solution was heated for 3 h of reaction. After the completion of the reaction, the reaction solution was added with 10 mL of water, adjusted to pH=6 with 3M dilute hydrochloric acid, and extracted with ethyl acetate (10 mL × 3). The organic phases were combined, washed with saturated sodium chloride solution (20 mL), dried over anhydrous sodium sulfate, filtered and concentrated under reduced pressure. The residue was purified by silica gel column chromatography to obtain 2-(3,5-dichloro-4-((5-cyclopropyl-6-oxo-1,6-dihydropyridazin-3-yl)oxy)phenyl)-6-(hydroxymeth yl)-1,2,4-triazine-3,5(2H,4H)-dione **11** (250 mg, white solid), yield: 95%. MS m/z (ESI): 437.95 [M+1]⁺. ¹H NMR (400 MHz, DMSO-*d₆*) δ 12.20 (s, 1H), 7.86 (s, 2H), 7.18 (s, 1H), 5.30 (t, *J* = 6.0 Hz, 1H), 4.40 (d, *J* = 6.0 Hz, 2H), 2.18-2.12 (m, 1H), 1.06-1.11 (m, 2H), 0.98-1.04 (m, 2H).

### Example 12

### 2-(3,5-dichloro-4-((5-cyclopropyl-6-oxo-1,6-dihydropyridazin-3-yl)oxy)phenyl)-6-(fluoromethyl )-1,2,4-triazine-3,5(2H,4H)-dione

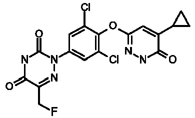

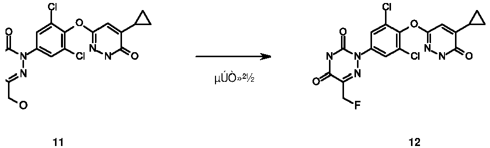

### Step 1

### 2-(3,5-dichloro-4-((5-cyclopropyl-6-oxo-1,6-dihydropyridazin-3-yl)oxy)phenyl)-6-(fluoromethyl )-1,2,4-triazine-3,5(2H,4H)-dione

2-(3,5-dichloro-4-((5-cyclopropyl-6-oxo-1,6-dihydropyridazin-3-yl)oxy)phenyl)-6-(hyd roxymethyl)-1,2,4-triazine-3,5(2H,4H)-dione **11** (100 mg, 0.22 mmol) was dissolved in 5 mL of dichloromethaneand and added with DAST (34 mg, 1.05 mmol) under ice bath, and the reaction solution was reacted at this temperature for 30 min. After the completion of the reaction, the reaction solution was added to 10 mL of ice water and extracted with dichloromethane (15 mL × 3). The organic phases were combined, washed with saturated sodium chloride solution, dried over anhydrous sodium sulfate, filtered and concentrated under reduced pressure. The residue was purified by preparative chromatography to obtain 2-(3,5-dichloro-4-((5-cyclopropyl-6-oxo-1,6-dihydropyridazin-3-yl)oxy)phenyl)-6-(fluoromethyl )-1,2,4-triazine-3,5(2H,4H)-dione **12** (26 mg, white solid), yield: 26%. MS m/z (ESI): 439.95 [M+1]⁺. ¹H NMR (400 MHz, DMSO-*d₆*) δ 12.20 (s, 1H), 7.81 (s, 2H), 7.18 (s, 1H), 5.35-5.23 (d, *J =* 48.0 Hz, 2H), 2.13-2.15 (m,1H), 1.09-1.24 (m, 2H), 1.01-1.07 (m, 2H).

### Example 13

### 2-(3,5-dichloro-4-((5-cyclobutyl-6-oxo-1,6-dihydropyridazin-3-yl)oxy)phenyl)-6-(hydroxymethy 1)-1,2,4-triazine-3,5(2H,4H)-dione

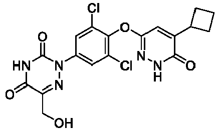

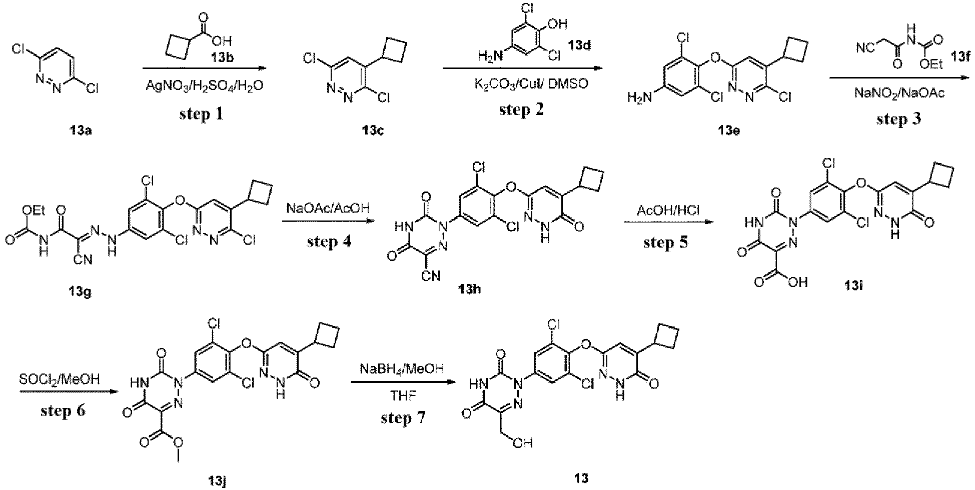

### Step 1

### 3,6-dichloro-4-cyclobutylpyridazine

3,6-dichloropyridazine **13a** (5.0 g, 33.6 mmol) was dissolved in a mixture of 168 mL of water and 7.4 mL of concentrated sulfuric acid, then added with silver nitrate (1.14 g, 6.72 mmol) followed by cyclobutyric acid **13b** (3.5 ml, 36.9 mmol), and slowly added with 65 mL of aqueous solution of ammonium persulfate (23 g, 100.6 mmol) dropwise at room temperature within half an hour. After the dropwise addition, the reaction solution was heated to 70°C for 0.5 h of reaction, then cooled to room temperature, adjusted to pH=7 with ammonia water and extracted with ethyl acetate (50 mL × 3). The organic phases were combined, dried over anhydrous sodium sulfate and filtered, and the filtrate was concentrated under reduced pressure. The resultant residue was purified by silica gel column chromatography to obtain3,6-dichloro-4-cyclobutylpyridazine **13c** (6.2 g, colorless oily liquid), yield: 91%. MS m/z (ESI): 204.0 [M+1]⁺.

### Step 2

### 3,5-dichloro-4-((6-chloro-5-cyclobutylpyridazin-3-yl)oxy)aniline

3,6-dichloro-4-cyclobutylpyridazine **13c** (5.23 g, 25.75 mmol) and 4-amino-2,6-dichlorophenol **5d** (4.58 g, 25.75 mmol) were dissolved in 50 mL of dimethyl sulfoxide, added with potassium carbonate (7.1 g, 51.50 mmol) followed by cuprous iodide (2.45 g, 12.88 mmol), heated to 90°C and stirred for 5 h. The reaction solution was cooled to room temperature, added with 100 mL of water and extracted with ethyl acetate (50 mL × 3). The organic phases were combined, washed with water (50 mL × 3), dried over anhydrous sodium sulfate and filtered, and the filtrate was concentrated under reduced pressure. The resultant residue was purified by silica gel column chromatography to obtain 3,5-dichloro-4-((6-chloro-5-cyclobutylpyridazin-3-yl)oxy)aniline **13e** (6.5 g, white solid), yield: 73%. MS m/z (ESI): 345.0 [M+1]⁺.

### Step 3

### (E)-ethyl(2-cyano-2-(2-(3,5-dichloro-4-((6-chloro-5-cyclobutylpyridazin-3-yl)oxy)phenyl)hydraz ine)acetyl)carbamate

3,5-dichloro-4-((6-chloro-5-cyclopentylpyridazin-3-yl)oxy)aniline **13e** (1.72 g, 5.0 mmol) was dissolved in 16 mL of acetic acid and 4 mL of water, cooled down to 0°C , and then added with 2 mL of concentrated hydrochloric acid. 1 mL of aqueous sodium nitrite (414 mg, solution 6.00 mmol) was slowly added dropwise. After the dropwise addition, the reaction solution was stirred for 10 min, then added with 5 mL of aqueous solution of sodium acetate (1.23 g, 15.0 mmol) followed by ethyl (2-cyanoacetic acid) carbamate **13f** (936 mg, 6.00 mmol). After ice bath was removed, and the reaction solution was warmed to room temperature for 1 h of reaction. 30 mL of water was added, and the solid was filtered and dried to obtain solid (E)-ethyl(2-cyano-2-(2-(3,5-dichloro-4-((6-chloro-5-cyclobutylpyridazin-3-yl)oxy)phenyl)hydraz ine)acetyl)carbamate **13g** (2.2 g, pale yellow solid), yield: 85%. MS m/z (ESI): 512.1 [M+1]⁺.

### Step 4

### 2-(3,5-dichloro-4-((6-chloro-5-cyclobutylpyridazin-3-yl)oxy)phenyl)-3,5-dioxo-2,3,4,5-tetrahydr o-1,2,4-triazine-6-carbonitrile

Compound (E)-ethyl(2-cyano-2-(2-(3,5-dichloro-4-((6-chloro-5-cyclobutylpyridazin-3-yl)oxy)phenyl)hydraz ine)acetyl)carbamate **13g** (1.53 g, 3.0 mmol) was dissolved in 30 mL of glacial acetic acid, then added with sodium acetate (792 mg, 6.0 mmol), heated to 120C , and stirred for 3 h. The reaction wolution was cooled to room temperature, added with 50 mL of water and extracted with ethyl acetate (30 mL × 3). The organic phases were combined, dried over anhydrous sodium sulfate and filtered, and the filtrate was concentrated under reduced pressure. The resultant residue was purified by silica gel column chromatography to obtain 2-(3,5-dichloro-4-((5-cyclobutyl-6-oxo-1,6-dihydropyridazin-3-yl)oxy)phenyl)-3,5-dioxo-2,3,4,5 -tetrahydro-1,2,4-triazine-6-carbonitrile **13h** (1.14 g, white solid), yield: 85%. MS m/z (ESI): 448.1 [M+1]⁺. ¹H NMR (400 MHz, DMSO-*d₆*) δ 13.28 (brs, 1H), 12.19 (s, 1H), 7.78 (s, 2H), 7.49 (d, *J* = 1.2 Hz, 1H), 3.48-3.62 (m, 1H), 2.23-2.33 (m, 2H), 1.88-2.02 (m, 1H), 1.75-1.86 (m, 1H).

### Step 5

### 2-(3,5-dichloro-4-((5-cyclobutyl-6-oxo-1,6-dihydropyridazin-3-yl)oxy)phenyl)-3,5-dioxo-2,3,4,5 -tetrahydro-1,2,4-triazine-6-carboxylic acid

2-(3,5-dichloro-4-((5-cyclobutyl-6-oxo-1,6-dihydropyridazin-3-yl)oxy)phenyl)-3,5-diox o-2,3,4,5-tetrahydro-1,2,4-triazine-6-carbonitrile **13h** (1.34 g, 3.0 mmol) was dissolved in 25 mL of glacial acetic acid, added with 5 mL of concentrated hydrochloric acid, and heated to 120°C for 5 h of reaction. After the completion of the reaction, the reaction solution was cooled to room temperature, added with 50 mL of water to dilute, stirred and filtered, and the solid was dried to obtain
2-(3,5-dichloro-4-((5-cyclobutyl-6-oxo-1,6-dihydropyridazin-3-yl)oxy)phenyl)-3,5-dioxo-2,3,4,5 -tetrahydro-1,2,4-triazine-6-carboxylic acid **13i** (1.3 g, pale yellow solid), yield: 93%. MS m/z (ESI): 467.0 [M+1]⁺.

### Step 6

### Methyl 2-(3,5-dichloro-4-((5-cyclobutyl-6-oxo-1,6-dihydropyridazin-3-yl)oxy)phenyl)-3,5-dioxo-2,3,4,5 -tetrahydro-1,2,4-triazine-6-carboxylate

2-(3,5-dichloro-4-((5-cyclobutyl-6-oxo-1,6-dihydropyridazin-3-yl)oxy)phenyl)-3,5-diox o-2,3,4,5-tetrahydro-1,2,4-triazine-6-carboxylic acid **13i** (466 mg, 1.0 mmol) was dissolved in 10 mL methanol, slowly added with 2 mL of thionyl chloride under ice bath. The reaction solution was heated to 80°C for 5 h of reaction. After the completion of the reaction, the reaction solution was concentrated under reduced pressure, and the residual solid was slurried with petroleum ether: ethyl acetate (20:1) and filtered. The solid was dried to obtain methyl 2-(3,5-dichloro-4-((5-cyclopentyl-6-oxo-1,6-dihydropyridazin-3-yl)oxy)phenyl)-3,5-dioxo-2,3,4, 5-tetrahydro-1,2,4-triazine-6-carboxylate **13j** (450 mg, pale yellow solid), yield: 93%. MS m/z (ESI): 481.1 [M+1]⁺.

### Step 7

### 2-(3,5-dichloro-4-((5-cyclobutyl-6-oxo-1,6-dihydropyridazin-3-yl)oxy)phenyl)-6-(hydroxymethy 1)-1,2,4-triazine-3,5(2H,4H)-dione

Methyl 2-(3,5-dichloro-4-((5-cyclobutyl-6-oxo-1,6-dihydropyridazin-3-yl)oxy)phenyl)-3,5-dioxo-2,3,4,5 -tetrahydro-1,2,4-triazine-6-carboxylate **13j** (450 mg, 0.93 mmol) was dissolved in 10 mL of tetrahydrofuran, then added with sodium borohydride (344 mg, 9.3 mmol), and heated to 60°C. Methanol was slowly added dropwise until no gas was generated, and the reaction solution was heated for 3 h of reaction. After the completion of the reaction, the reaction solution was added with 20 mL of water, adjusted to pH=6 with 3M dilute hydrochloric acid, and extracted with ethyl acetate (30 mL × 3). The organic phases were combined, washed with saturated sodium chloride solution (100 mL), dried over anhydrous sodium sulfate, filtered and concentrated under reduced pressure. The residue was purified by silica gel column chromatography to obtain 2-(3,5-dichloro-4-((5-cyclobutyl-6-oxo-1,6-dihydropyridazin-3-yl)oxy)phenyl)-6-(hydroxymethy 1)-1,2,4-triazine-3,5(2H,4H)-dione **13** (300 mg, white solid), yield: 71%. MS m/z (ESI): 452.0 [M+1]⁺. ¹H NMR (400 MHz, DMSO-*d₆*) δ 12.46 (brs, 1H), 12.15 (s,1H), 7.86 (s, 2H), 7.47 (d, *J* = 1.2 Hz, 1H), 5.30 (t, *J* = 6.4 Hz, 1H), 4.40 (d, *J* = 6.4 Hz, 2H), 3.48-3.61 (m, 1H), 2.22-2.28 (m, 2H), 2.07-2.16 (m, 2H), 1.97-2.02 (m, 1H), 1.76-1.85 (m, 1H).

### Example 14

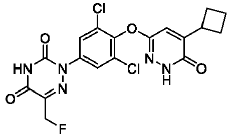

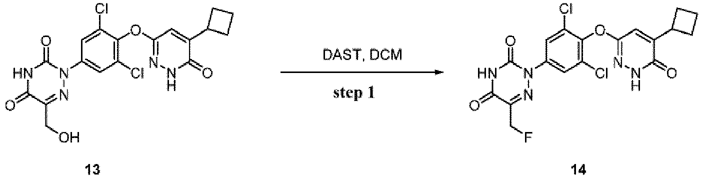

### Step 1

### 2-(3,5-dichloro-4-((5-cyclobutyl-6-oxo-1,6-dihydropyridazin-3-yl)oxy)phenyl)-6-(fluoromethyl)-1,2,4-triazine-3,5(2H,4H)-dione 14

2-(3,5-dichloro-4-((5-cyclobutyl-6-oxo-1,6-dihydropyridazin-3-yl)oxy)phenyl)-6-(hydr oxymethyl)-1,2,4-triazine-3,5(2H,4H)-dione **13** (200 mg, 0.416 mmol) was dissolved in 5 mL of dichloromethane and added with diethylaminosulfur trifluoride (0.55 ml, 4.16 mmol) at 0°C to react for 0.5 h. The reaction solution was concentrated under reduced pressure to remove the solvent, and purified by preparative chromatography to obtain 2-(3,5-dichloro-4-((5-cyclobutyl-6-oxo-1,6-dihydropyridazin-3-yl)oxy)phenyl)-6-(fluoromethyl)-1,2,4-triazine-3,5(2H,4H)-dione **14** (5.5 mg, white solid), yield: 2.8%. MS m/z (ESI): 454.0 [M+1]⁺. ¹H NMR (400 MHz, DMSO-*d₆*) δ 12.68 (brs, 1H), 12.17 (s, 1H) 7.81 (s, 2H), 7.49 (d, *J* = 1.2 Hz, 1H), 5.30 (d, *J* = 48.0 Hz, 2H), 3.52-3.62 (m, 1H), 2.11-2.23 (m, 2H), 1.96-2.05 (m, 1H), 1.74-1.84 (m, 1H).

### Example 15

### 2-(3,5-dichloro-4-((5-(2-fluoropropan-2-yl)-6-oxo-1,6-dihydropyridazin-3-yl)oxy)phenyl)-6-(hyd roxymethyl)-1,2,4-triazine-3,5(2H,4H)-dione

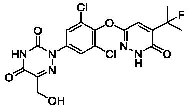

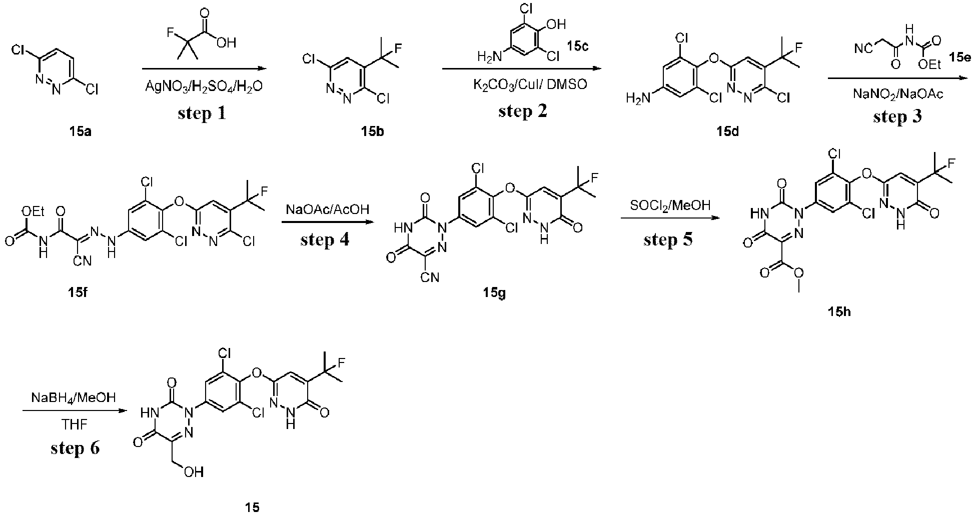

### Step 1

### 3,6-dichloro-4-(2-fluoropropan-2-yl)pyridazine

3,6-dichloropyridazine **15a** (5 g, 33.56 mmol) was dissolved in a mixture of 20 mL of water and 4.09 mL of concentrated sulfuric acid, added with silver nitrate (0.57 g, 3.35 mmol) followed by fluoroisobutyric acid **15b** (3.25 g, 28.56 mmol), and slowly added with 10 mL of aqueous solution of ammonium persulfate (15.31 g, 67.1 mmol) dropwise at room temperature within half an hour. After the dropwise addition, the reaction solution was heated to 80°C for 0.4 h of reaction, then cooled to room temperature, adjusted to pH=7 with ammonia water and extracted with ethyl acetate (25 mL × 3). The organic phases were combined, dried over anhydrous sodium sulfate and filtered, and the filtrate was concentrated under reduced pressure. The resultant residue was purified by silica gel column chromatography to obtain 3,6-dichloro-4-(2-fluoropropan-2-yl)pyridazine **15b** (3.3 g, white solid), yield: 47%. MS m/z (ESI): 209.0 [M+1]⁺.

### Step 2

### 3,5-dichloro-4-((6-chloro-5-(2-fluoropropan-2-yl)pyridazin-3-yl)oxy)aniline

3,6-dichloro-4-(2-fluoropropan-2-yl)pyridazine **15b** (3.3 g, 15.79 mmol) and 4-amino-2,6-dichlorophenol **15c** (3.65 g, 20.52 mmol) were dissolved in 40 mL of dimethyl sulfoxide, then added with potassium carbonate (4.36 g, 31.57 mmol), cuprous iodide (1.5 g, 7.89 mmol), heated to 90°C and stirred for 4 h. The reaction solution was cooled to room temperature, added with 30 mL of water and extracted with ethyl acetate (50 mL × 3). The organic phases were combined, dried over anhydrous sodium sulfate and filtered, and the filtrate was concentrated under reduced pressure. The resultant residue was purified by silica gel column chromatography to obtain 3,5-dichloro-4-((6-chloro-5-(2-fluoropropan-2-yl)pyridazin-3-yl)oxy)aniline **15d** (5.1 g, yellow solid), yield: 92%. MS m/z (ESI): 351.2 [M+1]⁺.

### Step 3

### (E)ethyl(Z)-(2-cyano-2-(2-(3,5-dichloro-4-((6-chloro-5-(2-fluoropropan-2-yl)pyridazine-3-yl)oxy )phenyl)hydrazino)acetylcarbamate

3,5-dichloro-4-((6-chloro-5-(2-fluoropropan-2-yl)pyridazin-3-yl)oxy)aniline **15d** (2 g, 5.7 mmol) was dissolved in 32 mL of acetic acid and 8 mL of water, cooled to 0°C, then added with 4 mL of concentrated hydrochloric acid, and then slowly added with 1 mL of aqueous solution of sodium nitrite (512 mg, 7.42 mmol) dropwise. After the dropwise addition, the reaction solution was stirred for 10 min at 0°C, then added with 5 mL of aqueous solution of sodium acetate (1.4 g, 17.11 mmol) followed by ethyl (2-cyanoacetic acid) carbamate **15e** (1.16 g, 7.42 mmol), removed of ice bath, and warmed to room temperature for 1 h of reaction. 30 mL of water was added, and the solid was filtered and dried to obtain solid (E)ethyl(Z)-(2-cyano-2-(2-(3,5-dichloro-4-((6-chloro-5-(2-fluoropropan-2-yl)pyridazine-3-yl)oxy )phenyl)hydrazino)acetylcarbamate **15f** (3.1 g, pale yellow solid), yield: 94.8%. MS m/z (ESI): 517.0 [M+1]⁺.

### Step 4

### 2-(3,5-dichloro-4-((5-(2-fluoropropan-2-yl)-6-oxo-1,6-dihydropyridazin-3-yl)oxy)phenyl)-3,5-di oxo-2,3,4,5-tetrahydro-1,2,4-triazine-6-carbonitrile

Compound (E)ethyl(Z)-(2-cyano-2-(2-(3,5-dichloro-4-((6-chloro-5-(2-fluoropropan-2-yl)pyridazine-3-yl)oxy )phenyl)hydrazino)acetylcarbamate **15f** (2.75 g, 5.31 mmol) was dissolved in 30 mL of glacial acetic acid, then added with sodium acetate (871 mg, 10.6 mmol), heated to 120°C and stirred for 4 h. The reaction solution was cooled to room temperature, added with 50 mL of water and extracted with ethyl acetate (30 mL × 3). The organic phases were combined, dried over anhydrous sodium sulfate and filtered, and the filtrate was concentrated under reduced pressure. The resultant residue was purified by silica gel column chromatography to obtain 2-(3,5-dichloro-4-((6-chloro-5-(2-fluoropropan-2-yl)pyridazin-3-yl)oxy)phenyl)-3,5-dioxo-2,3,4, 5-tetrahydro-1,2,4-triazine-6-carbonitrile **15g** (1.8 g, yellow solid), yield: 71.8%. MS m/z (ESI): 452.9 [M+1]⁺. ¹H NMR (400 MHz, DMSO-*d₆*) δ 12.52 (brs, 1H), 7.81 (s, 2H), 7.51 (s, 1H), 1.72 (d, *J* = 24.0 Hz, 6H).

### Step 5

### Methyl 2-(3,5-dichloro-4-((5-(2-fluoropropan-2-yl)-6-oxo-1,6-dihydropyridoazin-3-yl)oxy)phenyl)-3,5-d ioxo-2,3,4,5-tetrahydro-1,2,4-triazine-6-carboxylate

2-(3,5-dichloro-4-((6-chloro-5-(2-fluoropropan-2-yl)pyridazin-3-yl)oxy)phenyl)-3,5-dio xo-2,3,4,5-tetrahydro-1,2,4-triazine-6-carbonitrile **15g** (300 mg, 0.62 mmol) was dissolved in 4 mL of methanol, added slowly with 2 mL of thionyl chloride under ice bath, and heated to 80°C for 4 h of reaction. After the completion of the reaction, the reaction solution was concentrated under reduced pressure, added with 15 mL of water and extracted with ethyl acetate (30 mL × 3). The organic phases were combined, dried over anhydrous sodium sulfate and filtered, and the filtrate was concentrated under reduced pressure. The resultant residue was purified by silica gel column chromatography to obtain methyl 2-(3,5-dichloro-4-((5-(2-fluoropropan-2-yl)-6-oxo-1,6-dihydropyridoazin-3-yl)oxy)phenyl)-3,5-d ioxo-2,3,4,5-tetrahydro-1,2,4-triazine-6-carboxylate **15h** (300 mg, pale yellow solid), yield: 93%. MS m/z (ESI): 485.9 [M+1]⁺.

### Step 6

### 2-(3,5-dichloro-4-((5-(2-fluoropropan-2-yl)-6-oxo-1,6-dihydropyridazin-3-yl)oxy)phenyl)-6-(hyd roxymethyl)-1,2,4-triazine-3,5(2H,4H)-dione

Methyl 2-(3,5-dichloro-4-((5-(2-fluoropropan-2-yl)-6-oxo-1,6-dihydropyridoazin-3-yl)oxy)phenyl)-3,5-d ioxo-2,3,4,5-tetrahydro-1,2,4-triazine-6-carboxylate **15h** (100 mg, 0.205 mmol) was dissolved in 6 mL of tetrahydrofuran, then added with sodium borohydride (40 mg, 1.03 mmol), and heated to 60°C. Methanol was slowly added dropwise until no gas was generated, and the reaction solution was heated for 3 h of reaction. After the completion of the reaction, the reaction solution was added with 20 mL of water, adjusted to pH=6 with 3M dilute hydrochloric acid, and extracted with ethyl acetate (30 mL × 3). The organic phases were combined, washed with saturated sodium chloride solution (100 mL), dried over anhydrous sodium sulfate, filtered and concentrated under reduced pressure. The residue was purified by silica gel column chromatography to obtain 2-(3,5-dichloro-4-((5-(2-fluoropropan-2-yl)-6-oxo-1,6-dihydropyridazin-3-yl)oxy)phenyl)-6-(hyd roxymethyl)-1,2,4-triazine-3,5(2H,4H)-dione **15** (90 mg, white solid), yield: 95.5%. MS m/z (ESI): 457.95 [M+1]⁺. ¹H NMR (400 MHz, DMSO-*d₆*) δ 12.50 (s, 1H), 12.46 (brs, 1H), 7.88 (s, 2H), 7.50 (s, 1H), 5.32 (t, *J* = 4.0 Hz, 1H), 4.40 (d, *J* = 4.0 Hz, 2H), 1.72 (d, *J* = 24.0 Hz, 6H).

### Example 16

### 3-(3,5-dichloro-4-((5-(2-fluoropropan-2-yl)-6-oxo-1,6-dihydropyridazin-3-yl)oxy)phenyl)-6-(fluo romethyl)-1,2,4-triazine-3,5(2H,4H)-dione

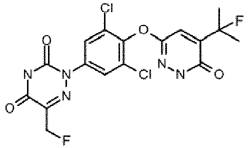

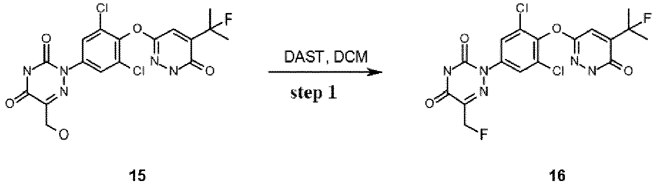

### Step 1

### 2-(3,5-dichloro-4-((5-(2-fluoropropan-2-yl)-6-oxo-1,6-dihydropyridazin-3-yl)oxy)phenyl)-6-(fluo romethyl)-1,2,4-triazine-3,5(2H,4H)-dione

2-(3,5-dichloro-4-((5-(2-fluoropropan-2-yl)-6-oxo-1,6-dihydropyridazin-3-yl)oxy)phen yl)-6-(hydroxymethyl)-1,2,4-triazine-3,5(2H,4H)-dione **15** (90 mg, 0.20 mmol) was dissolved in 5 mL of dichloromethane and added with diethylaminosulfur trifluoride (158 mg, 0.98 mmol) at 0°C to react for 2 min. The reaction solution was concentrated under reduced pressure to remove the solvent, and purified by preparative chromatography to obtain 2-(3,5-dichloro-4-((5-(2-fluoropropan-2-yl)-6-oxo-1,6-dihydropyridazin-3-yl)oxy)phenyl)-6-(fluo romethyl)-1,2,4-triazine-3,5(2H,4H)-dione **16** (16.0 mg, white solid), yield: 18%. MS m/z(ESI): 459.9 [M+1]⁺. ¹H NMR (400 MHz, DMSO-*d₆*) δ 12.68 (brs, 1H), 12.49 (s, 1H) 7.83 (s, 2H), 7.50 (s, 1H), 5.30 (d, *J* = 44.0 Hz , 2H) 1.71(d, *J* = 24Hz, 6H).

### Example 17

### 2-(3,5-dichloro-4-((6-oxo-5-(prop-1-en-2-yl)-1,6-dihydropyridazin-3 -yl)oxy)phenyl)-6-(hydroxy methyl)-1,2,4-triazine-3,5(2H,4H)-dione

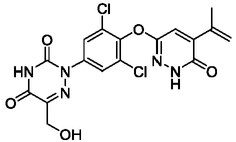

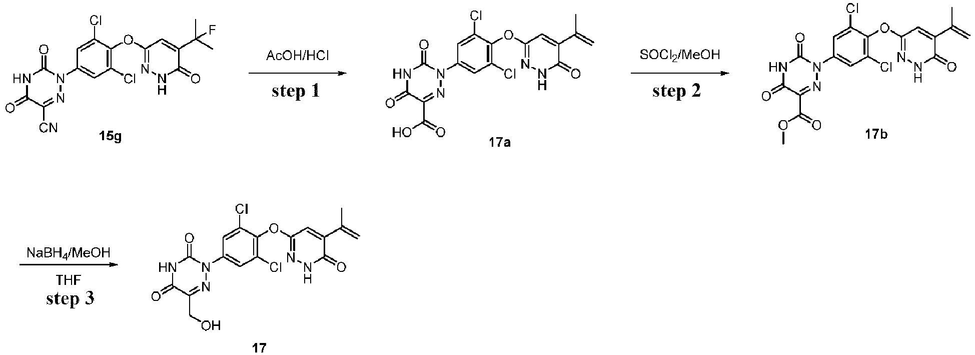

### Step 1

### 2-(3,5-dichloro-4-((6-oxo-5-(prop-1-en-2-yl)-1,6-dihydropyridazin-3-yl)oxy)phenyl)-3,5-dioxo-2 ,3,4,5-tetrahydro-1,2,4-triazine-6-carboxylic acid

2-(3,5-dichloro-4-((5-(2-fluoropropan-2-yl)-6-oxo-1,6-dihydropyridazin-3-yl)oxy)phen yl)-3,5-dioxo-2,3,4,5-tetrahydro-1,2,4-triazine-6-carbonitrile **15a** (1.5 g, 3.31 mmol) was dissolved in acetic acid (25 ml), then slowly added with hydrochloric acid (5 ml) dropwise, and stirred at 120°C for 4 h. The reaction solution was cooled to room temperature and extracted with ethyl acetate (25 mL × 3). The organic phases were combined, dried over anhydrous sodium sulfate and filtered, and the filtrate was concentrated under reduced pressure. The resultant residue was purified by silica gel column chromatography to obtain 2-(3,5-dichloro-4-((6-oxo-5-(prop-1-en-2-yl)-1,6-dihydropyridazin-3-yl)oxy)phenyl)-3,5-dioxo-2 ,3,4,5-tetrahydro-1,2,4-triazine-6-carboxylic acid **17a** (1.0 g, yellow solid), yield: 67%. MS m/z (ESI): 451.9 [M+1]⁺.

### Step 2

### Methyl 2-(3,5-dichloro-4-((6-oxo-5-(prop-1-en-2-yl)-1,6-dihydropyridazin-3-yl)oxy)phenyl)-3,5-dioxo-2 ,3,4,5-tetrahydro-1,2,4-triazine-6-carboxylate

2-(3,5-dichloro-4-((6-oxo-5-(prop-1-en-2-yl)-1,6-dihydropyridazin-3-yl)oxy)phenyl)-3, 5-dioxo-2,3,4,5-tetrahydro-1,2,4-triazine-6-carboxylic acid **17a** (300 mg, 0.663 mmol) was dissolved in 4 mL of methanol, added slowly with 2 mL of thionyl chloride under ice bath, and the reaction solution was heated to 70°C for 4 h of reaction. After the completion of the reaction, the reaction solution was concentrated under reduced pressure, added with 15 mL of water and extracted with ethyl acetate (30 mL × 3). The organic phases were combined, dried over anhydrous sodium sulfate and filtered, and the filtrate was concentrated under reduced pressure. The resultant residue was purified by silica gel column chromatography to obtain methyl 2-(3, 5-dichloro-4-((6-oxo-5-(prop-1-en-2-yl)-1,6-dihydropyridazin-3-yl)oxy)phenyl)-3,5-dioxo-2 ,3,4,5-tetrahydro-1,2,4-triazine-6-carboxylate **17b** (250 mg , pale yellow solid), yield: 81%. MS m/z (ESI): 466.0 [M+1]⁺.

### Step 3

### 2-(3,5-dichloro-4-((6-oxo-5-(prop-1-en-2-yl)-1,6-dihydropyridazin-3 -yl)oxy)phenyl)-6-(hydroxy methyl)-1,2,4-triazine-3,5(2H,4H)-dione

Methyl 2-(3,5-dichloro-4-((6-oxo-5-(prop-1-en-2-yl)-1,6-dihydropyridazin-3-yl)oxy)phenyl)-3,5-dioxo-2 ,3,4,5-tetrahydro-1,2,4-triazine-6-carboxylate **17b** (100 mg, 0.214 mmol) was dissolved in 6 mL of tetrahydrofuran, then added with sodium borohydride (41 mg, 1.7 mmol), and heated to 60°C. Methanol was slowly added dropwise until no gas was generated, and the reaction solution was heated for 4 h of reaction. After the completion of the reaction, the reaction solution was added with 20 mL of water, adjusted to pH=6 with 3M dilute hydrochloric acid, and extracted with ethyl acetate (30 mL × 3). The organic phases were combined, washed with saturated sodium chloride solution (100 mL), dried over anhydrous sodium sulfate, filtered and concentrated under reduced pressure. The residue was purified by silica gel column chromatography to obtain 2-(3,5-dichloro-4-((6-oxo-5-(prop-1-en-2-yl)-1,6-dihydropyridazin-3 -yl)oxy)phenyl)-6-(hydroxy methyl)-1,2,4-triazine-3,5(2H,4H)-dione **17** (90.0 mg, white solid), yield: 96%. MS m/z (ESI): 437.9 [M+1]⁺. ¹H NMR (400 MHz, DMSO-*d₆*) δ 12.46 (s, 1H), 12.36 (s, 1H), 7.86 (s, 2H), 7.56 (s, 1H), 6.50(s, 1H), 5.56(s, 1H), 5.30 (t, *J* = 4.0 Hz, 1H), 4.40 (d, *J* = 4.0 Hz, 2H), 2.11 (s, 3H).

### Example 18

### 2-(3,5-dichloro-4-((6-oxo-5-(prop-1-en-2-yl)-1,6-dihydropyridazin-3-yl)oxy)phenyl)-6-(fluorome thyl)-1,2,4-triazine-3,5(2H,4H)-dione

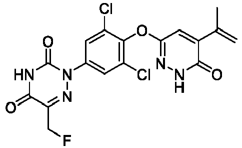

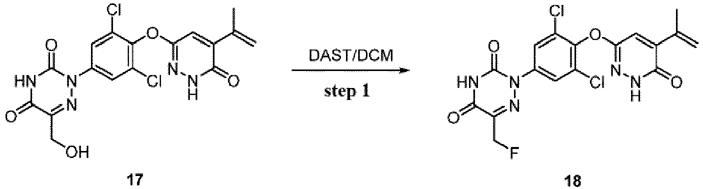

### Step 1

### 2-(3,5-dichloro-4-((6-oxo-5-(prop-1-en-2-yl)-1,6-dihydropyridazin-3-yl)oxy)phenyl)-6-(fluorome thyl)-1,2,4-triazine-3,5(2H,4H)-dione

2-(3,5-dichloro-4-((6-oxo-5-(prop-1-en-2-yl)-1,6-dihydropyridazin-3-yl)oxy)phenyl)-6-(hydroxymethyl)-1,2,4-triazine-3,5(2H,4H)-dione **17** (90 mg, 0.20 mmol) was dissolved in 5 mL of dichloromethane and added with diethylaminosulfur trifluoride (158 mg, 0.98 mmol) at 0°C to react for 2 min. The reaction solution was concentrated under reduced pressure to remove the solvent, and purified by preparative chromatography to obtain 2-(3,5-dichloro-4-((6-oxo-5-(prop-1-en-2-yl)-1,6-dihydropyridazin-3-yl)oxy)phenyl)-6-(fluorome thyl)-1,2,4-triazine-3,5(2H,4H)-dione **18** (4.0 mg, white solid), yield: 4.4%. MS m/z(ESI): 439.9 [M+1]⁺. ¹H NMR (400 MHz, DMSO-*d₆*) δ 12.46 (s, 1H), 12.36 (s, 1H) 7.83 (s, 2H), 7.56 (s, 1H), 6.50 (s, 1H), 5.56 (s, 1H), 5.30 (d, *J* = 48.0 Hz ,2H), 2.11(s, 3H).

### Example 19

### 6-cyclopropyl-2-(3,5-dichloro-4-((5-isopropyl-6-oxo-1,6-dihydropyridazin-3-yl)oxy)phenyl)-1,2, 4-triazine-3,5(2H,4H)-dione

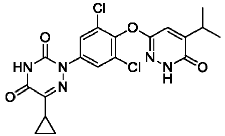

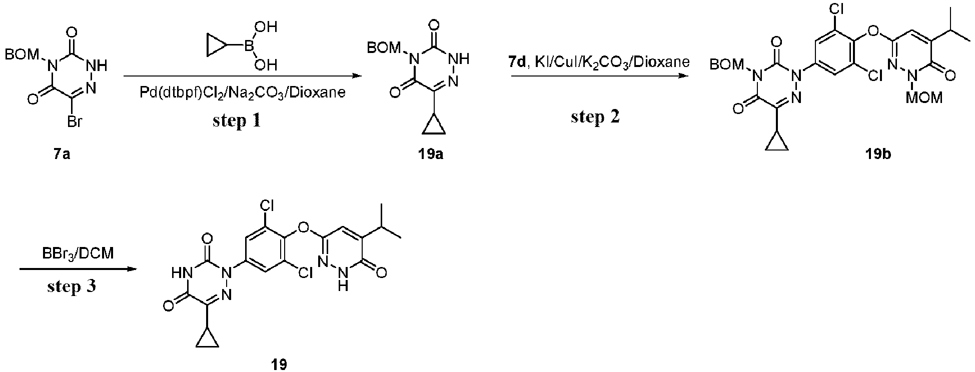

### Step 1

### 4-((benzyloxy)methyl)-6-cyclopropyl-1,2,4-triazine-3,5(2H,4H)-dione

4-((benzyloxy)methyl)-6-bromo-1,2,4-triazine-3,5(2H,4H)-dione **7a** (500 mg, 1.61 mmol) was added to 20 mL of dioxane, then added with cyclopropylboronic acid (690 mg, 8.0 mmol), [1,1'-bis(di-tert-butylphosphino)ferrocene]dichloropalladium (210 mg, 0.32 mmol) and 2 mol/L aqueous sodium carbonate solution (5 mL), replaced with nitrogen three times, and then reacted at 80°C for 7 h. The reaction solution was concentrated under reduced pressure to remove the solvent, added with 1 mol/L hydrochloric acid solution (50 mL) and extracted with ethyl acetate (20 mL × 3). The organic phases were combined, dried over anhydrous sodium sulfate, filtered and concentrated. The residue was purified by column chromatography (by rinsing with petroleum ether: ethyl acetate from 1:0 to 3:1) to obtain 4-((benzyloxy)methyl)-6-cyclopropyl-1,2,4-triazine-3,5(2H,4H)-dione **19a** (120 mg, white solid), yield: 27%. MS m/z (ESI): 274.0 [M+1]⁺.

### Step 2

### 4-((benzyloxy)methyl)-6-cyclopropyl-2-(3,5-dichloro-4-((5-isopropyl-1-(methoxymethyl)-6-oxy-1,6-dihydropyridazin-3-yl)oxy)phenyl)-1,2,4-triazine-3,5(2H,4H)-dione

4-((benzyloxy)methyl)-6-cyclopropyl-1,2,4-triazine-3,5(2H,4H)-dione **19a** (76 mg, 0.28 mmol) was dissolved in 2 mL of dioxane, then added with 6-(2,6-dichloro-4-iodophenoxy)-4-isopropyl-2-(methoxymethyl)pyridazine-3(2H)-one **7d** (130 mg, 0.28 mmol) followed by trans-(1R,2R)-N,N'-dimethyl 1,2-cyclohexanediamine (8 mg, 0.055 mmol) and potassium iodide (46 mg, 0.28 mmol), cuprous iodide (53 mg, 0.28 mmol), and potassium carbonate (77 mg, 0.56 mmol), and then microwaved at 130°C for 2 h of reaction. The reaction solution was added with 10 mL of ethyl acetate and 10 mL of water, and the layers were separated. The aqueous phase was extracted with 10 mL of ethyl acetate once, and the organic phases were combined, washed with 10 mL of saturated brine once, dried over anhydrous sodium sulfate, filtered and concentrated. The residue was purified by column chromatography (by rinsing with petroleum ether: ethyl acetate from 1:0 to 5:1) to obtain 4-((benzyloxy)methyl)-6-cyclopropyl-2-(3,5-dichloro-4-((5-isopropyl-1-(methoxymethyl)-6-oxy-1,6-dihydropyridazin-3-yl)oxy)phenyl)-1,2,4-triazine-3,5(2H,4H)-dione **19b** (150 mg, colorless oil), yield: 88 %. MS m/z (ESI): 614.0 [M+1]⁺.

### Step 3

### 6-cyclopropyl-2-(3,5-dichloro-4-((5-isopropyl-6-oxo-1,6-dihydropyridazin-3-yl)oxy)phenyl)-1,2, 4-triazine-3, 5 (2H,4H)-dione

4-((benzyloxy)methyl)-6-cyclopropyl-2-(3,5 -dichloro-4-((5-isopropyl-1-(methoxymethy l)-6-oxy-1,6-dihydropyridazin-3-yl)oxy)phenyl)-1,2,4-triazine-3,5(2H,4H)-dione **19b** (75 mg, 0.12 mmol) was dissolved in 2 mL of dichloromethane. Under nitrogen protection, boron tribromide (0.36 mL, 1 mol/L) was added to react overnight at room temperature. 1 mL of methanol was added to quench the reaction, and then the solvent was removed by concentration under reduced pressure. Then 1 mL of methanol and 1 mL of concentrated sulfuric acid were added, and the reaction was carried out at 100°C 1 h. After the completion of the reaction, the reaction solution was cooled to room temperature, added dropwise to ice water, adjusted to pH=5~6 with saturated aqueous potassium carbonate solution, and extracted with ethyl acetate twice (20 mL × 2). The organic phases were combined, washed with 10 mL of saturated brine once, dried over anhydrous sodium sulfate, filtered and concentrated. The residue was purified by preparative chromatography to obtain 6-cyclopropyl-2-(3,5-dichloro-4-((5-isopropyl-6-oxo-1,6-dihydropyridazin-3-yl)oxy)phenyl)-1,2, 4-triazine-3,5(2H,4H)-dione **19** (20.0 mg, white solid ), yield: 36%. MS m/z (ESI): 450.0[M+1] ⁺. ¹H NMR (400 MHz, DMSO-*d₆*) δ 12.39 (brs, 1H), 12.20 (s, 1H), 7.76 (s, 2H), 7.44 (s, 1H), 3.03-3.10 (m, 1H), 2.15-2.23 (m, 1H), 1.20 (d, *J* = 4.0 Hz, 6H), 0.88-1.00 (m, 4H).

### Example 20

### 2-(3,5-dichloro-4-((5-isopropyl-6-oxo-1,6-dihydropyridazin-3-yl)oxy)phenyl)-6-hydroxy-1,2,4-tr iazine-3,5(2H,4H)-dione

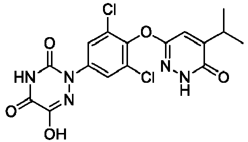

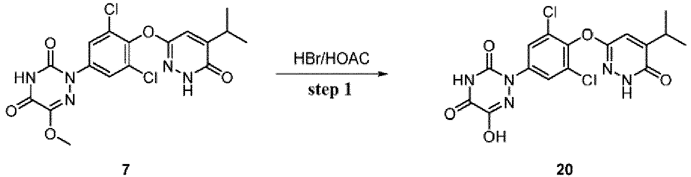

### Step 1

### 2-(3,5-dichloro-4-((5-isopropyl-6-oxo-1,6-dihydropyridazin-3-yl)oxy)phenyl)-6-hydroxy-1,2,4-tr iazine-3,5(2H,4H)-dione

2-(3,5-dichloro-4-((5-isopropyl-6-oxo-1,6-dihydropyridazin-3-yl)oxy)phenyl)-6-methox y-1,2,4-triazine-3,5(2H,4H)-dione 7 (25 mg, 0.057 mmol) was dissolved in 1 mL of hydrobromic acid and 1 mL of acetic acid, microwaved at 100°C for 8 h of reaction, and concentrated under reduced pressure to remove the solvent. The residue was separated by preparative chromatography to obtain 2-(3,5-dichloro-4-((5-isopropyl-6-oxo-1,6-dihydropyridazin-3-yl)oxy)phenyl)-6-hydroxy-1,2,4-tr iazine-3,5(2H,4H)-dione **20** (0.5 mg, white solid), yield: 2%. MS m/z (ESI): 426.2 [M+1]⁺.

### Example 21 THRβ binding assay

Experimental method: In vitro analysis of the agonistic effect of the compounds on THRβ was performed using a time-resolved fluorescence resonance energy transfer co-activating peptide recruitment assay. The assay used Eu-anti-GST antibody, biotin-SRC2-2 co-activating peptide, streptavidin-d2, RXRα and GST-tagged THRβ-LBD. The Eu-anti-GST antibody indirectly labeled THRβ-LBD by binding to GST tag. Streptavidin-d2 indirectly labeled the SRC2-2 co-activating peptide by binding to biotin tag. In the presence of RXRα, THRβ-LBD can form a heterodimer THPβ-LBD/RXRα with it. The agonist bound to THPβ-LBD/RXRα and caused a conformational change of THKβ-LBD, thereby increasing the recruitment capacity of the heterodimer to the SRC2-2 co-activating peptide. Furthermore, the resulting decreased distance between the d2-labeled SRC2-2 co-activating peptide and the Eu-anti-GST antibody increased the THR-FRET signal. The agonistic ability of a compound can be assessed based on the effect of different concentrations of the compound on THRβ activity.

The detailed procedure is as follows.
a. 100X reference compound or compound was prepared with DMSO and diluted in 1:3.
b. 100X serially diluted reference compound or compound was diluted to 4X with 1X reaction buffer and added to the assay plate.
c. Mixed solutions of 4X THPβ-LBD and 4X RXRα were prepared with 1X reaction buffer and added to the assay plate.
d. Mixed solutions of 2X biotin-SRC2-2, 2X Eu-anti-GST and 2X streptavidin-d2 were prepared with 1X reaction buffer and added to the assay plate.
e. The plates were centrifuged at 1000 rpm for 1 min and incubated for 4 h at room temperature shielded from light.
f. The 665 nm and 615 nm fluorescence signal values were read on the EnVision 2104 plate reader and the Ratio665nm/615nm was calculated.

Experimental results: see Table 1

**Table 1: Test results of THRβ binding assay**

| Example No. | EC₅₀(nM) | Emax(%) |
|---|---|---|
| 1 | 737 | 109.3 |
| 2 | 5 | 92.76 |
| 3 | 23 | 100.6 |
| 4 | 159 | 123.8 |
| 9 | 101 | 108.4 |
| 10 | 94 | 109.9 |
| 12 | 71 | 105.4 |
| 14 | 42 | 117.4 |
| 16 | 163.6 | 111.3 |
| 18 | 23.6 | 124.5 |
| 19 | 7249 | 77.7 |
| 20 | >10000 | / |
| ^{∗}Control compound 1 | 0.6 | 97.3 |
| ^{∗}Control compound 2 | 204 | 105.4 |

| | | |
|---|---|---|
| ^{∗}Control compound 1 was T3; Control compound 2 was WO2007009913 Example 8 (Compound 31). | | |

### Example 22 THRα binding assay

Experimental method: In vitro analysis of the agonistic effect of compounds on THRα was performed using a similar method to the THRβ binding assay in Example 11, except that THRα was used instead of THRβ.

Experimental results: see Table 2

**Table 2: Test results of THRα binding assay**

| Example No. | EC₅₀(nM) | Emax(%) |
|---|---|---|
| 2 | 190 | 106.6 |
| 3 | 140 | 123.2 |
| 9 | 4260 | 122.7 |
| 10 | 1410 | 112.2 |
| 12 | 210 | 127 |
| 14 | 230 | 123.8 |
| ^{∗}Control compound 1 | 0.2 | 91.4 |
| ^{∗}Control compound 2 | 2690 | 111.4 |

| | | |
|---|---|---|
| ^{∗}Control compound 1 was T3; Control compound 2 was WO2007009913 Example 8 (Compound 31). | | |

### Example 23 In vitro liver microsome stability assay

### Experimental method:

### (1) Solution preparation

The test substance and the positive control verapamil were respectively dissolved in DMSO to 10 mM as a stock solution, and the above 10 mM stock solution was diluted with 70% aqueous acetonitrile solution to a concentration of 0.25 mM

An NADPH regeneration system containing 6.5 mM NADP, 16.5 mM G-6-P, 3 U/mL G-6-PDH, and 3.3 mM magnesium chloride was prepared.

The stop solution was an acetonitrile solution containing tolbutamide and propranolol (both internal standards).

Phosphate buffer was 100 mM K3PO4 (pH=7.4) buffer containing 3.3 mM MgCl2.

The liver microsome incubation system was in 100 mM phosphate buffer containing 0.2 mg/mL liver microsomal protein and 1 µM test substance/positive control.

### (2) Incubation process

80 µL of the mixture was transferred from the incubation system to 400 µL of stop solution to precipitate proteins, and 20 µL of NADPH regeneration system was added after vortexing as a 0 min sample point.

130 µL of NADPH regeneration system was added to the remaining 520 µL of protein drug mixture and mixed well to start incubation. The final incubation system was 650 µL, containing 0.2 mg/mL liver microsomal protein, 1 µM test substance/positive control, 1.3 mM NADP, 3.3 mM G-6-P and 0.6 U/mL G-6-PDH.

The mixed system was incubated with slow shaking in a 37°C water bath, and at 5, 10, 30, and 60 min, respectively, 100 µL of incubation solution was transferred into each well of a new 96-well plate containing 400 µL of stop solution and mixed well to precipitate proteins (4000 × g, centrifugation at 4°C for 15 min).

100 µL of the supernatant was diluted with water at a ratio of 1:2 and analyzed by LC-MS/MS.

Experimental results: see Table 3.

**Table 3: Results of in vitro liver microsome stability assay**

| Species | Parameter | Example 2 | Control Compound 2 | Verapamil |
|---|---|---|---|---|
| Human | T_{1/2} (min) | 1028.5 | 310.7 | 27.2 |
| | CL (µL/min/mg) | 3.4 | 11.2 | 127.3 |
| Monkey | T_{1/2} (min) | 535.0 | 297.9 | 5.6 |
| | CL (µL/min/mg) | 6.5 | 11.6 | 618.3 |
| Canine | T_{1/2} (min) | 264.1 | 119.8 | 24.4 |
| | CL ( µL/min/mg) | 13.1 | 28.9 | 142.2 |
| Rat | T_{1/2} (min) | 739.9 | 765.8 | 14.0 |
| | CL (µL/min/mg) | 4.7 | 4.5 | 246.7 |
| Mouse | T_{1/2} (min) | 186.9 | 203.6 | 10.1 |
| | CL (µL/min/mg) | 18.5 | 17.0 | 342.5 |

The above description of the examples is only used to facilitate understanding of the method and core concept of the present disclosure. It should be noted that, for those of ordinary skill in the art, several improvements and modifications can also be made without departing from the principle of the present invention, which also fall within the protection scope of the claims of the present disclosure.

## Claims

1. A 1,2,4-triazine-3,5-dione compound and pharmaceutically acceptable salt thereof, wherein the 1,2,4-triazine-3,5-dione compound has a structure represented by formula (I), 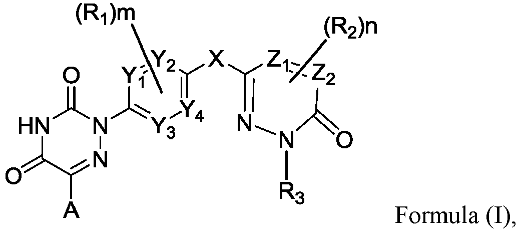
wherein, A is substituted or unsubstituted C1~C15 alkyl, substituted or unsubstituted C1~C15 alkoxy, substituted or unsubstituted C3~C18 cycloalkyl, substituted or unsubstituted C6~C20 aryl, substituted or unsubstituted C5~C20 heteroaryl, or substituted or unsubstituted C3~C18 heterocyclyl, hydroxyl or halogen;
X is substituted methylene, -O- or -S-,
Y₁, Y₂, Y₃, Y₄, Z₁ and Z₂ are independently selected from N and CR,
R₁, R₂, R₃ and R are independently selected from hydrogen, substituted or unsubstituted C1~C15 alkyl, substituted or unsubstituted C1~C15 alkoxy, substituted or unsubstituted C3~C18 cycloalkyl, and substituted or unsubstituted C2~C15 unsaturated hydrocarbyl, halogen or cyano; and
m and n are each an integer of 0-3.

2. The 1,2,4-triazine-3,5-dione compound and pharmaceutically acceptable salt thereof according to claim 1, wherein the substituent in the substituted C1~C15 alkyl, substituted C1~C15 alkoxy, substituted C3~C18 cycloalkyl, substituted C6~C20 aryl, substituted C5~C20 heteroaryl, substituted C3~C18 heterocyclyl and substituted C2~C15 unsaturated hydrocarbyl is hydroxyl, fluorine, chlorine, bromine, iodine or amino.

3. The 1,2,4-triazine-3,5-dione compound and pharmaceutically acceptable salt thereof according to claim 1, wherein A is methyl, ethyl, n-propyl, isopropyl, n-butyl, isobutyl, tert-butyl, n-pentyl, isopentyl, n-hexyl, n-heptyl, hydroxymethyl, hydroxyethyl, 2-hydroxypropyl, 3-hydroxypropyl, 2-hydroxybutyl, 2-hydroxypentyl, fluoromethyl, difluoromethyl, trifluoromethyl, 1-fluoroethyl, 1-fluoropropyl, 1-fluoroisopropyl, 1-fluorobutyl, 1,1-difluoroethyl, 1,1-difluoropropyl, 1,1-difluorobutyl, methoxy, ethoxy, propoxy, butoxy, pentoxy, cyclopropyl, cyclobutyl, cyclopentyl, cyclohexyl, phenyl, naphthyl, anthracenyl, pyridyl, pyrrolyl, furanyl, pyranyl, hydroxy, fluorine, chlorine, bromine or iodine.

4. The 1,2,4-triazine-3,5-dione compound and pharmaceutically acceptable salt thereof according to claim 1, wherein R₁, R₂, R₃ and R are independently selected from hydrogen, methyl, ethyl, n-propyl, isopropyl, n-butyl, isobutyl, tert-butyl, n-pentyl, isopentyl, n-hexyl, n-heptyl, hydroxymethyl, hydroxyethyl, 2-hydroxypropyl, 3-hydroxypropyl, 2-hydroxybutyl, 2-hydroxypentyl, fluoromethyl, difluoromethyl, trifluoromethyl, 1-fluoroethyl, 1-fluoropropyl, 1-fluoroisopropyl, 1-fluorobutyl, 1,1-difluoroethyl, 1,1-difluoropropyl, 1,1-difluorobutyl, methoxy, ethoxy, propoxy, butoxy, pentoxy, cyclopropyl, cyclobutyl, cyclopentyl, cyclohexyl, phenyl, naphthyl, anthracenyl, pyridyl, pyrrolyl, furyl, pyranyl, 1-methylvinyl, propenyl, butenyl, hydroxyl, fluorine, chlorine, bromine and iodine.

5. The 1,2,4-triazine-3,5-dione compound and pharmaceutically acceptable salt thereof according to claim 1, wherein the structure represented by formula (I) is specifically:

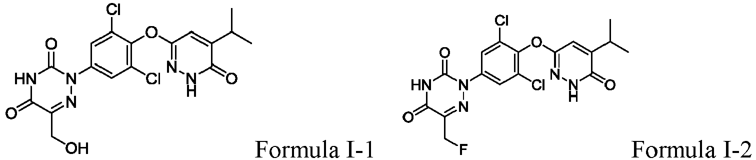

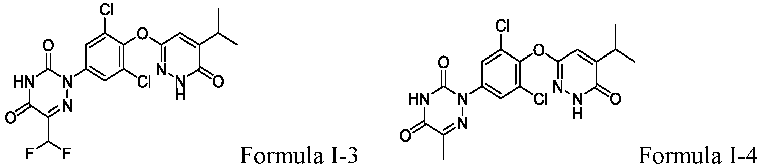

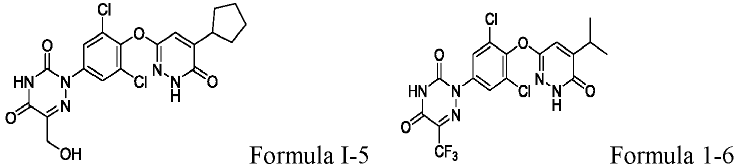

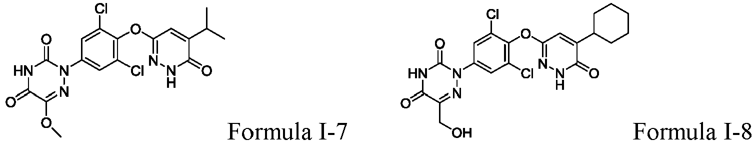

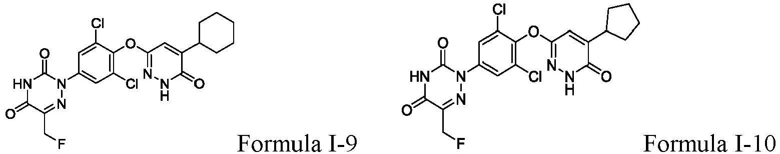

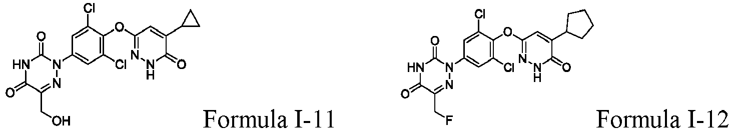

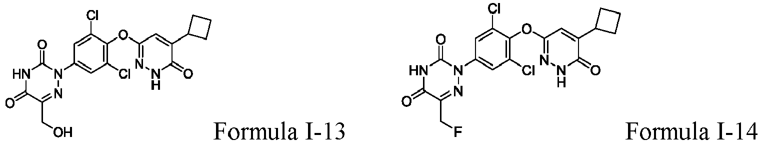

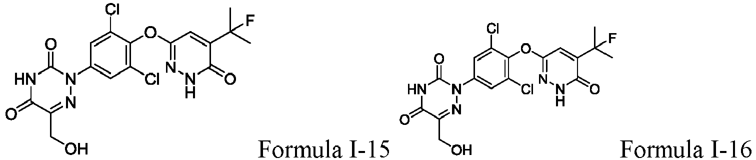

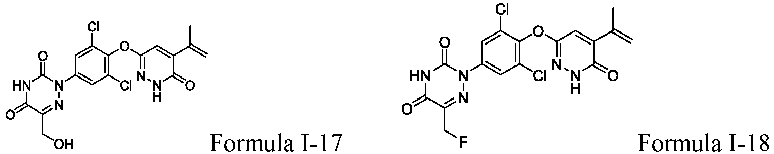

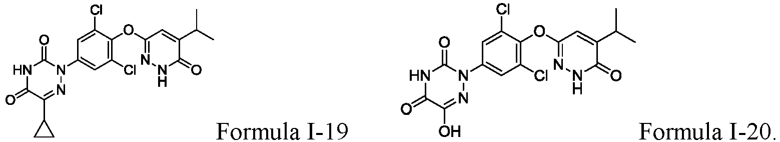

6. A method of preparing a 1,2,4-triazine-3,5-dione compound and pharmaceutically acceptable salt thereof, comprising:
converting a compound of formula 1b into a compound of formula 1c, and then converting the compound of formula 1c into a 1,2,4-triazine-3,5-dione compound having a structure represented by formula (I), 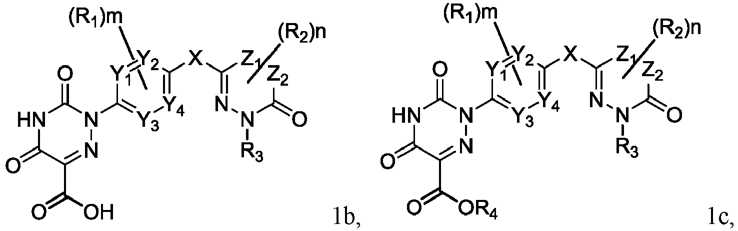 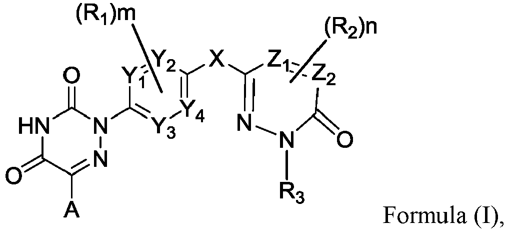
wherein, A is substituted or unsubstituted C1~C15 alkyl, substituted or unsubstituted C1~C15 alkoxy, substituted or unsubstituted C3~C18 cycloalkyl, substituted or unsubstituted C6~C20 aryl, substituted or unsubstituted C5~C20 heteroaryl, or substituted or unsubstituted C3~C18 heterocyclyl, hydroxyl or halogen;
X is substituted methylene, -O- or -S-,
Y₁, Y₂, Y₃, Y₄, Z₁ and Z₂ are independently selected from N and CR,
R₁, R₂, R₃ and R are independently selected from hydrogen, substituted or unsubstituted C1~C15 alkyl, substituted or unsubstituted C1~C15 alkoxy, substituted or unsubstituted C3~C18 cycloalkyl, and substituted or unsubstituted C2~C15 unsaturated hydrocarbyl, halogen or cyano;
m and n are each an integer of 0-3; and
R₄ is C1~C6 alkyl.

7. The preparation method according to claim 6, wherein the formula 1b is prepared by:
hydrolyzing a compound of formula 1a under an acidic condition to obtain a compound of formula 1b; 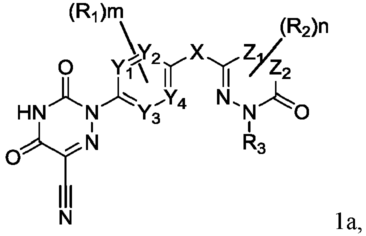
wherein X is substituted methylene, -O- or -S-,
Y₁, Y₂, Y₃, Y₄, Z₁ and Z₂ are independently selected from N and CR,
R₁, R₂, R₃ and R are independently selected from hydrogen, substituted or unsubstituted C1~C15 alkyl, substituted or unsubstituted C1~C15 alkoxy, substituted or unsubstituted C3~C18 cycloalkyl, and substituted or unsubstituted C2~C15 unsaturated hydrocarbyl, halogen or cyano; and
m and n are each an integer of 0-3.

8. A method of preparing a 1,2,4-triazine-3,5-dione compound and pharmaceutically acceptable salt thereof, comprising:
reacting a compound of formula 4d with a compound of formula 4e to obtain a 1,2,4-triazine-3,5-dione compound having a structure represented by formula (I), 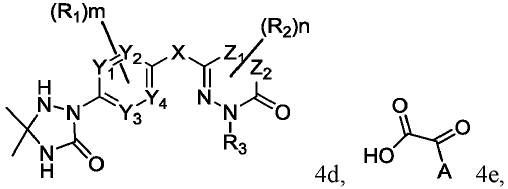 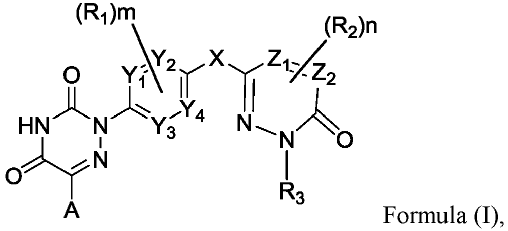
wherein, A is substituted or unsubstituted C1~C15 alkyl, substituted or unsubstituted C1~C15 alkoxy, substituted or unsubstituted C3~C18 cycloalkyl, substituted or unsubstituted C6~C20 aryl, substituted or unsubstituted C5~C20 heteroaryl, or substituted or unsubstituted C3~C18 heterocyclyl, hydroxyl or halogen;
X is substituted methylene, -O- or -S-,
Y₁, Y₂, Y₃, Y₄, Z₁ and Z₂ are independently selected from N and CR,
R₁, R₂, R₃ and R are independently selected from hydrogen, substituted or unsubstituted C1~C15 alkyl, substituted or unsubstituted C1~C15 alkoxy, substituted or unsubstituted C3~C18 cycloalkyl, and substituted or unsubstituted C2~C15 unsaturated hydrocarbyl, halogen or cyano; and
m and n are each an integer of 0-3.

9. The preparation method according to claim 8, wherein the compound of formula 4d is prepared by:
converting a compound of formula 4c into a compound of formula 4d, 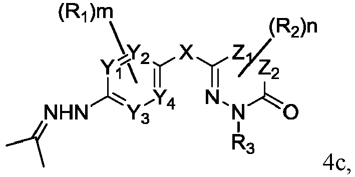
wherein X is substituted methylene, -O- or -S-,
Y₁, Y₂, Y₃, Y₄, Z₁ and Z₂ are independently selected from N and CR,
R₁, R₂, R₃ and R are independently selected from hydrogen, substituted or unsubstituted C1~C15 alkyl, substituted or unsubstituted C1~C15 alkoxy, substituted or unsubstituted C3~C18 cycloalkyl, and substituted or unsubstituted C2~C15 unsaturated hydrocarbyl, halogen or cyano; and
m and n are each an integer of 0-3.

10. Use of the 1,2,4-triazine-3,5-dione compound and pharmaceutically acceptable salt thereof according to any one of claims 1 to 5 in the manufacture of a THR agonist.
